(19) Europäisches Patentamt
European Patent Office
Office européen des brevets

(11) **EP 2 403 398 B1**

(12) **EUROPÄISCHE PATENTSCHRIFT**

(45) Veröffentlichungstag und Bekanntmachung des Hinweises auf die Patenterteilung:
**11.07.2018 Patentblatt 2018/28**

(21) Anmeldenummer: **10711559.4**

(22) Anmeldetag: **05.03.2010**

(51) Int Cl.:
*A61B 5/0402* (2006.01)     *A61B 5/1455* (2006.01)
*A61B 5/00* (2006.01)     *A61B 5/024* (2006.01)
*A61B 5/053* (2006.01)

(86) Internationale Anmeldenummer:
**PCT/EP2010/001374**

(87) Internationale Veröffentlichungsnummer:
**WO 2010/099969 (10.09.2010 Gazette 2010/36)**

(54) **DIAGNOSTISCHE MESSVORRICHTUNG**

DIAGNOSTIC MEASURING DEVICE

DISPOSITIF POUR MESURE DIAGNOSTIQUE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **05.03.2009 DE 102009011381**

(43) Veröffentlichungstag der Anmeldung:
**11.01.2012 Patentblatt 2012/02**

(73) Patentinhaber: **Flore, Ingo**
**44141 Dortmund (DE)**

(72) Erfinder:
• **KIM, Yoon Ok**
**58239 Schwerte (DE)**

• **CHO, Ok Kyung**
**58239 Schwerte (DE)**

(74) Vertreter: **Isfort, Olaf**
**Schneiders & Behrendt PartmbB**
**Rechts- und Patentanwälte**
**Huestraße 23**
**(Kortumkarree)**
**44787 Bochum (DE)**

(56) Entgegenhaltungen:
WO-A1-90/04352     WO-A1-2005/048831
WO-A1-2008/061788     US-A- 6 041 247
US-A1- 2005 131 282     US-A1- 2008 275 317
US-B1- 6 230 035

EP 2 403 398 B1

**Beschreibung**

Die Erfindung betrifft eine diagnostische Messvorrichtung zur nicht-invasiven Erfassung von wenigstens einem physiologischen Parameter von Körpergewebe, mit einer optischen Messeinheit, die wenigstens eine Strahlungsquelle zur Bestrahlung des zu untersuchenden Körpergewebes und wenigstens einen Strahlungssensor zur Detektion der von dem Körpergewebe gestreuten und/oder transmittierten Strahlung umfasst, wobei die wenigstens eine Strahlungsquelle in einem Hohlreflektor angeordnet ist.

Die Versorgung des Körpergewebes mit Sauerstoff gehört bekanntlich zu den wichtigsten Vitalfunktionen des Menschen. Aus diesem Grund sind oximetrische Diagnosemodalitäten heutzutage von großer Bedeutung in der Medizin. Routinemäßig werden so genannte Pulsoximeter eingesetzt. Die diagnostische Sensoreinheit derartiger Pulsoximeter umfasst typischerweise eine optische Messeinheit mit zwei Lichtquellen, die rotes bzw. infrarotes Licht unterschiedlicher Wellenlänge in das Körpergewebe einstrahlen. Das Licht wird im Körpergewebe gestreut und teilweise absorbiert. Das gestreute Licht wird schließlich mittels eines Lichtsensors in Form einer geeigneten Photozelle (Photodiode) detektiert. Die Intensität des mittels des Lichtsensors detektierten, gestreuten Lichts variiert in Abhängigkeit davon, wie stark das untersuchte Körpergewebe von sauerstoffreichem, bzw. sauerstoffarmem Blut durchblutet ist. Dementsprechend ist mittels des Pulsoximeters die Sauerstoffsättigung des Blutes messbar. Die bekannten Pulsoximeter sind außerdem in der Lage, ein plethysmographisches Signal, d. h. ein Volumenpulssignal zu erzeugen, das die während des Herzschlags veränderliche Blutmenge in dem von dem Pulsoximeter erfassten Mikrogefäßsystem wiedergibt (sog. Photoplethysmographie).

Es ist beispielsweise aus der US 6 041 247 eine Messvorrichtung der eingangs genannten Art bekannt. Auch hier werden mehrere Lichtquellen mit unterschiedlichen Wellenlängen bevorzugt.

Der vorliegenden Erfindung liegt die Aufgabe zugrunde, eine diagnostische Messvorrichtung zur nicht-invasiven Bestimmung von physiologischen Parametern bereit zu stellen, die kompakt baut und kostengünstig herstellbar ist.

Diese Aufgabe löst die Erfindung ausgehend von einer Messvorrichtung der eingangs angegebenen Art dadurch, dass die Abstrahlung des Lichts der wenigstens einen Strahlungsquelle in der Weise erfolgt, dass Lichtstrahlen mit unterschiedlichen räumlichen Abstrahlcharakteristiken das untersuchte Körpergewebe bestrahlen, wobei am Hohlreflektor wenigstens zwei Strahlungsaustrittsöffnungen vorgesehen sind und an wenigstens einer dieser Austrittsöffnungen eine Optik zur Veränderung der räumlichen Abstrahlcharakteristik der aus dem Hohlreflektor emittierten Strahlung angebunden ist.

Die Ausgestaltung der erfindungsgemäßen Messvorrichtung ermöglicht einen besonders kompakten und robusten Aufbau. Der Hohlreflektor kann z.B. nach Art einer sog. Ulbricht-Kugel ausgebildet sein, wobei die Geometrie des Hohlreflektors im Sinne der Erfindung nicht auf eine Kugelform beschränkt ist. Bei einer Ulbricht-Kugel handelt es sich um einen innen diffus reflektierend beschichteten Hohlkörper. Die im Inneren reflektierte und gestreute Strahlung der Lichtquelle ist nahezu ideal diffus, d.h., dass die Richtcharakteristik der Strahlungsquelle, z.B. der Leuchtdiode, weitgehend aufgehoben ist. Der Hohlreflektor muss dabei nicht zwingend hohl sein, sondern kann auch aus transparentem Material hergestellt sein, das an der Außenseite beschichtet ist. Die eigentliche Strahlungsquelle (z.B. der LED-Chip) kann dabei in das Material des Hohlreflektors eingebettet sein, wobei die Stromversorgung über eine aus dem Hohlreflektor heraus geführte Drahtverbindung erfolgen kann. Auf diese Weise lässt sich die optische Messeinheit miniaturisieren. Der Hohlreflektor kann als sehr kompaktes Bauteil zu geringen Kosten in großen Stückzahlen vorgefertigt werden.

Gemäß einer alternativen Ausführungsform kann der Hohlreflektor durch ein Chipgehäuse gebildet sein, das in sich die Strahlungsquelle und/oder den Strahlungssensor aufnimmt, wobei die Strahlungsquelle und der Strahlungssensor jeweils wenigstens einen Halbleiterkörper umfassen, der über im Inneren des Chipgehäuses verlaufende elektrische Leiterbahnen (z.B. eines so genannten Leadframes) und/oder über Bond-Drahtverbindungen kontaktiert ist. Dabei weist das Chipgehäuse an seiner Oberseite Durchtrittsöffnungen zum Durchtritt der von der Strahlungsquelle emittierten Strahlung in das zu untersuchenden Körpergewebes und/oder zum Durchtritt der von dem Körpergewebe gestreuten und/oder transmittierten Strahlung auf. Das Chipgehäuse bildet gleichsam die Ummantelung der Halbleiterkörper (der Chips) der Strahlungsquelle (LED) und/oder des Strahlungssensors (Photodiode). Außerdem sind an dem Chipgehäuse die Anschlussstellen (Leads, Pins oder Balls) angeordnet. Damit dient das Chipgehäuse dazu, die Halbleiterkörper z.B. auf einer Leiterplatte der Messvorrichtung zu befestigen und die Halbleiterkörper mit einer Schaltung auf der Leiterplatte zu verbinden. Das Chipgehäuse bewirkt einen Schutz der Halbleiterkörper. Die Anschlüsse der Halbleiterkörper werden z.B. mittels Bonddraht an ein Zwischenmaterial gebondet (angeschlossen). Dieses Zwischenmaterial kann ein gestanztes Blech (Leadframe) oder eine kleine Platine als Substrat sein. Der Anschluss an die Leiterplatte der Messvorrichtung erfolgt schließlich über die externen Anschlussstellen des Chipgehäuses. Nach der Befestigung und Verdrahtung der Halbleiterkörper auf dem Zwischenmaterial werden sie zweckmäßigerweise durch unterschiedliche Materialien (Plastik, Keramik, Metall) hermetisch gegenüber Umwelteinflüssen geschützt. Dabei werden an der Oberseite des Chipgehäuses die Durchtrittsöffnungen für den Lichtein- und/oder -austritt angeordnet. Diese Öffnungen können mittels durchsichtigem Plastik (z.B. Epoxidharz) oder Quarzglas geschlossen sein, so dass die Halbleiterkörper nicht direkt der Umwelt ausgesetzt sind. Gemäß der Erfindung fungiert das Chipgehäuse gleichzeitig als Hohlreflektor. Hierzu kann das Chipgehäuse an seinen inneren Wandungen mit einem diffus reflektierenden Material beschichtet sein. Die im Inneren ggf. mehrfach reflektierte und gestreute Strahlung der Lichtquelle ist, ähnlich wie bei dem Einsatz der oben beschriebenen Ulbricht-Kugel, nahezu ideal diffus, d.h., dass die Richtcharakteristik der Strahlungsquelle, z.B. der Leuchtdiode, weitgehend aufgehoben ist. Die Strahlung der Strahlungsquelle verlässt somit das Chipgehäuse unter einem sehr großen Abstrahlwinkel, von mehr als 90°, vorzugsweise von mehr als 100°, was mit einer an das (miniaturisierte) Chipgehäuse angebundenen, herkömmlichen Optik nicht möglich wäre.

Gemäß einer bevorzugten Ausgestaltung der Messvorrichtung ist eine EKG-Einheit zur Erfassung eines EKG-Signals über zwei oder mehr EKG-Elektroden vorgesehen, wobei an der Gehäuseoberfläche des Sensorgehäuses wenigstens eine EKG-Elektrode der EKG-Einheit angeordnet ist. Weiterhin ist eine bioelektrische Impedanzmesseinheit vorgesehen, wobei an der Gehäuseoberfläche des Sensorgehäuses wenigstens eine Einspeise- oder Messelektrode der Impedanzmesseinheit angeordnet ist, in der Weise, dass die EKG-Elektrode und die Einspeise- oder Messelektrode die Hautoberfläche in dem von der optischen Messeinheit erfassten Bereich des Körpergewebes berühren.

Durch die erfindungsgemäße Integration einer optischen Messeinheit und einer EKG-Einheit wird eine kompakte Anordnung geschaffen, die eine Vielzahl von diagnostischen Messwerten liefert. Diese können einzeln oder in Kombination ausgewertet werden, um schnell und zuverlässig aussagekräftige Informationen zum Gesundheitszustand des untersuchten Patienten zu erhalten. Die kompakte Messvorrichtung kann als vollständig funktionsfähiges Teil kostengünstig in großen Stückzahlen vorgefertigt und in Diagnosegeräte verschiedenster Art integriert werden. Die eigentliche Messung kann besonders einfach und komfortabel durchgeführt werden. Hierzu wird die Oberfläche des Sensorgehäuses mit der Haut im Bereich des zu untersuchenden Körpergewebes in Kontakt gebracht, was z.B. durch Auflegen eines Fingers des Patienten auf die Gehäuseoberfläche der Sensoreinheit erfolgen kann. Die optische Messung, die EKG-Ableitung und die Impedanzmessung erfolgen dann gleichzeitig über die die Sensoreinheit berührende Hautstelle.

Die EKG-Einheit der erfindungsgemäßen Sensoreinheit dient zur Erfassung eines EKG-Signals über zwei oder mehr EKG-Elektroden. Hierdurch wird der Funktionsumfang der erfindungsgemäßen Sensoreinheit gegenüber herkömmlichen Systemen vorteilhaft erweitert. Die erfindungsgemäße Sensoreinheit ermöglicht es, pulsoximetrische Signale und EKG-Signale kombiniert zu erfassen und auszuwerten. Zweckmäßigerweise ist hierzu eine Auswertungseinheit zur Auswertung des zeitlichen Verlaufs der optisch gemessenen Volumenpulssignale und der EKG-Signale vorgesehen. Diese Auswertungseinheit kann integraler Bestandteil der Messvorrichtung sein. Ebenso kann vorgesehen sein, dass die Auswertungseinheit von der Messvorrichtung separat ist, wobei die Messsignale über eine geeignete Datenverbindung an die Auswertungseinheit übertragen werden. Mittels einer geeigneten Programmsteuerung ist die Auswertungseinheit z.B. dazu in der Lage, die R-Zacken in dem EKG-Signal automatisch zu erkennen. Damit wird automatisch der exakte Zeitpunkt des Herzschlags ermittelt. Weiterhin ist die Auswertungseinheit aufgrund geeigneter Programmsteuerung dazu in der Lage, die Maxima in dem Volumenpulssignal zu erkennen. Anhand der Maxima in dem Volumenpulssignal ist der Zeitpunkt des Eintreffens einer bei einem Herzschlag ausgelösten Pulswelle an dem von der Sensoreinheit erfassten peripheren Messort feststellbar. Somit kann schließlich der zeitliche Abstand zwischen einer R-Zacke in dem EKG-Signal und dem darauf folgenden Maximum in dem Volumenpulssignal ermittelt werden. Dieser zeitliche Abstand ist ein Maß für die so genannte Pulswellengeschwindigkeit. Auf der Basis der Pulswellengeschwindigkeit kann einerseits eine Aussage über den Blutdruck getroffen werden. Eine Verkürzung der Pulswellengeschwindigkeit geht nämlich mit einer Erhöhung des Blutdrucks einher, während eine Verlängerung der Pulswellengeschwindigkeit auf eine Blutdruckerniedrigung schließen lässt. Eine exakte Bestimmung des Blutdrucks aus der Pulswellengeschwindigkeit ist allerdings nicht möglich, es können nur Tendenzen angegeben werden. Weiterhin ist die Pulswellengeschwindigkeit von der Dichte des Blutes und insbesondere von der Elastizität der Blutgefäßwandungen (beispielsweise der Aorta) abhängig. Aus der Elastizität der Blutgefäße kann wiederum auf eine ggf. vorliegende Arteriosklerose geschlossen werden. Es können in diese Auswertung auch die Absolutwerte der Herzfrequenz, die Herzfrequenzvariabilität und entsprechende Arrhythmien des Herzens einbezogen werden. So können automatisch Arrhythmien wie Sinus Tachycardia, Sinus Bradycardia, Sinus Arrest und so genannte Escape Beats festgestellt werden. Anhand des EKG-Signals können außerdem Aussagen über die zeitliche Dauer der Vorhofkontraktion des Herzens bei einem Herzschlag, die zeitliche Dauer der Herzkammerkontraktion sowie die Dauer der Relaxation der Herzkammer usw. festgestellt werden. Außerdem sind Vordiagnosen bezüglich so genannter Blocks in der Leitung der elektrischen Erregungssignale am Herzen (AV-Block, Bundle Branch-Block usw.) und auch bezüglich Durchblutungsstörungen oder Infarkten möglich. Weitere Irregularitäten im Pulsverlauf sind anhand des Volumenpulssignals feststellbar.

Die Erfindung basiert u. a. auf der Erkenntnis, dass durch die Kombination verschiedener Diagnosemodalitäten in einer einzigen Messvorrichtung die Möglichkeit eröffnet wird, metabolische Parameter zu bestimmen.

Gemäß der Erfindung ist daher eine herkömmliche (optische) Oximetrieeinheit nicht nur mit einer EKG-Einheit, sondern auch mit einer bioelektrischen Impedanzmesseinheit in einer einzigen Messvorrichtung kombiniert. Aus den mittels der bioelektrischen Impedanzmesseinheit gewonnenen Messsignalen kann z.B. die Zusammensetzung des untersuchten Körpergewebes bestimmt werden. Auf dieser Grundlage kann dann, vorzugsweise mittels einer geeigneten programmgesteuerten Auswertungseinheit, die mit den Messeinheiten der erfindungsgemäßen Messvorrichtung verbunden ist, aus den oximetrischen Signalen der Sensoreinheit die kapillare Sauerstoffsättigung im Gewebe ermittelt werden. Die arterielle Sauerstoffsättigung ($SaO_2$) und die venöse Sauerstoffsättigung ($SvO_2$) bestimmen abhängig von der Art des untersuchten Gewebes die kapillare (arteriovenöse) Sauerstoffsättigung ($StO_2$). Es gilt:

$$K * SvO_2 + (1 - K) * SaO_2 = StO_2,$$

wobei K ein gewebeabhängiger Korrekturfaktor ist, der vom Volumenverhältnis von Arterien zu Venen im untersuchten Gewebe abhängt. Im Mittel liegt dieser Wert etwas unter 0,5. Der für das jeweilige Gewebe maßgebliche Wert kann gemäß der Erfindung durch bioelektrische Impedanzmessung ermittelt werden, um dann aus der obigen Formel die venöse Sauerstoffsättigung zu bestimmen. Die erfindungsgemäße Sensoreinheit kann genutzt werden, um die Durchblutung V, d. h. die durchblutungsbedingte Volumenschwankung des untersuchten Körpergewebes zu bestimmen. Nach der Beziehung

$$VO_2 = V * (SaO_2 - SvO_2)$$

kann dann schließlich der lokale Sauerstoffverbrauch $VO_2$ berechnet werden, der ein Maß für die metabolische Aktivität am Messort darstellt.

Für die bioelektrische Impedanzmessung sind Einspeise- oder Messelektroden an der Gehäuseoberfläche des Sensorgehäuses angeordnet, so dass gleichzeitig mit der oximetrischen und der EKG-Messung die Bioimpedanzmessung erfolgen kann. Dabei wird derselbe Bereich des Körpergewebes, nämlich dort, wo der Patient die Oberfläche des Sensorgehäuses berührt, von sämtlichen Messmodalitäten gleichzeitig erfasst.

Gemäß der Erfindung sind an der Oberfläche des Sensorgehäuses wenigstens eine EKG-Elektrode und wenigstens eine Einspeise- oder Messelektrode der Impedanzmesseinheit angeordnet. Die andere EKG-Elektrode und ggf. eine weitere Einspeise- oder Messelektrode der Impedanzmesseinheit sind zweckmäßigerweise so angeordnet, dass der Patient alle Elektroden mit verschiedenen Extremitäten berühren kann, beispielsweise mit jeder Hand jeweils eine der Elektroden.

Gemäß einer vorteilhaften Ausgestaltung umfasst die Messvorrichtung einen integrierten Temperatur- oder Wärmesensor. Dieser kann zur Bestimmung der lokalen Wärmeproduktion genutzt werden. Im einfachsten Fall ist der Temperatursensor (z.B. ein NTC-Element) zur Messung der Oberflächentemperatur der Haut am Messort ausgebildet. Vorzugsweise ist mittels des Wärmesensors eine orts-, zeit- und tiefenaufgelöste Wärmemessung am Messort möglich. Anhand des Wärmeaustauschs kann auf die lokale Stoffwechselaktivität zurückgeschlossen werden. Außerdem ist der Wärmesensor zur Bestimmung der lokalen Durchblutung geeignet. Bezüglich näherer Hintergrundinformationen zur Wärmemessung wird auf die Veröffentlichung von Nitzan et al. verwiesen (Meir Nitzan, Boris Khanokh, "Infrared Radiometry of Thermally Insulated Skin for the Assessment of Skin Blood Flow", Optical Engineering 33, 1994, No. 9, S. 2953 bis 2956). Insgesamt liefert der Wärmesensor Daten, die mit Vorteil zur Bestimmung von metabolischen Parametern genutzt werden können.

Besonders vorteilhaft ist die erfindungsgemäße Kombination der vorgenannten Messverfahren, nämlich der Oximetrie, der EKG-Messung, der Temperatur- bzw. Wärmemessung und der bioelektrischen Impedanzmessung. Sämtliche Messsignale können durch einen geeigneten Algorithmus ausgewertet und kombiniert werden. Durch die Kombination der verschiedenen Messmodalitäten wird eine hohe Effektivität, Redundanz und Zuverlässigkeit bei der Erkennung von pathologischen Veränderungen erreicht. Sämtliche Parameter können mit Vorteil zu einem globalen Index zusammengefasst werden, der für den Benutzer leicht interpretierbar ist und ihm einen direkten und fundierten Hinweis auf seinen allgemeinen Gesundheitszustand gibt.

Die Kombination der verschiedenen Messmodalitäten, die in der erfindungsgemäßen Messvorrichtung, wie oben beschrieben, zusammengefasst sind, ist weiterhin vorteilhaft, weil dadurch eine nicht-invasive indirekte Messung der Glukosekonzentration möglich ist. Die Bestimmung des Blutglukosespiegels mittels der erfindungsgemäßen Vorrichtung wird im Folgenden näher erläutert:

Der Metabolismus des menschlichen Körpers ist im Normalzustand, d. h. in Ruhe und in der so genannten thermoneutralen Zone, im Wesentlichen durch den Glukosehaushalt bestimmt. Daher kann die Glukosekonzentration in den Zellen des Körpergewebes in diesem Normalzustand als reine Funktion der Wärmeproduktion und des Sauerstoffverbrauchs beschrieben werden. Es gilt:

$$[Glu] = f_1(\Delta T, VO_2) ,$$

wobei [Glu] für die Glukosekonzentration steht. Die Wärmeproduktion $\Delta T$ kann mittels des Wärmesensors der erfindungsgemäßen Sensoreinheit z.B. aus der Differenz zwischen der arteriellen Temperatur und der Temperatur, welche die Hautoberfläche bei perfekter thermischer Isolierung erreichen würde, bestimmt werden ($\Delta T = T_\infty - T_{Arterie}$). $f_1(\Delta T, VO_2)$ gibt die funktionale Abhängigkeit der Glukosekonzentration von der Wärmeproduktion und vom Sauerstoffverbrauch an. Der Sauerstoffverbrauch ergibt sich, wie oben beschrieben, aus dem Unterschied zwischen venöser und arterieller Sauerstoffsättigung und der Durchblutung. Zur Bestimmung der Glukosekonzentration während bzw. direkt nach der Nahrungsaufnahme muss jedoch ein Korrekturterm berücksichtigt werden, der den Anteil des Fettstoffwechsels am Energiehaushalt wiedergibt. Es gilt dann:

$$[Glu] = f_1(\Delta T, VO_2) + X * f_2(\Delta T, VO_2)$$

X ist ein Faktor, der nach der Nahrungsaufnahme negativ ist. Dabei hängt X von der Zusammensetzung der aufgenommenen Nahrung ab. Insbesondere ist X davon abhängig, in welchem Verhältnis Fett und Kohlenhydrate am Metabolismus beteiligt sind. Der Faktor X lässt sich, wie oben beschrieben, anhand des zeitlichen Verlaufs der Pulswellengeschwindigkeit bestimmen. X ist 0, wenn reine Kohlenhydrate oder direkt Glukose aufgenommen werden. Der Betrag von X steigt an, je größer der Anteil von Fett an der aufgenommenen Nahrung ist. Zur Bestimmung des Korrekturfaktors X aus dem zeitlichen Verlauf der Pulswellengeschwindigkeit, der Blutdruckamplitude und/oder des Pulses wird normalerweise eine Eichung zur Anpassung an den jeweiligen Benutzer der Vorrichtung erforderlich sein. $f_2(\Delta T, VO_2)$ gibt für den Fettstoffwechsel die funktionale Abhängigkeit der Glukosekonzentration von der Wärmeproduktion und vom Sauerstoffverbrauch an.

Die erfindungsgemäße Messvorrichtung kann somit zur Bestimmung der lokalen Glukosekonzentration aus dem lokalen Sauerstoffverbrauch und der lokalen Wärmeproduktion verwendet werden. Hierzu weist die Messvorrichtung die geeigneten Messmodalitäten auf. Die Ermittlung des Sauerstoffverbrauchs, kann, wie oben erläutert, durch die Kombination der Oximetrie mit der bioelektrischen Impedanzmessung erfolgen. Zur Ermittlung der Wärmeproduktion ist dann noch zusätzlich der erwähnte Wärmesensor erforderlich. Um schließlich die Glukosekonzentration nach dem oben angegebenen funktionalen Zusammenhang berechnen zu können, sollte noch der Korrekturfaktor X, beispielsweise aus dem zeitlichen Verlauf der Pulswellengeschwindigkeit, ermittelt werden. Dies kann, wie ebenfalls oben erläutert, durch kombinierte Messung von EKG-Signalen und plethysmographischen Signalen erfolgen. Zur Bestimmung der Glukosekonzentration sind also zweckmäßigerweise in der erfindungsgemäßen Messvorrichtung ein Pulsoximeter, eine EKG-Einheit, eine bioelektrische Impedanzmesseinheit sowie ein Wärmesensor kombiniert.

Die zuvor skizzierte Methode erlaubt zunächst nur eine Bestimmung der intrazellulären Glukosekonzentration. Mit der Blutglukosekonzentration besteht vereinfacht der folgende Zusammenhang:

$$[Glu]_{Zelle} = a + b * \ln(c * [Glu]_{Blut})$$

Die Konstanten a, b und c hängen von der individuellen Physiologie des untersuchten Patienten ab. Diese Parameter können durch entsprechende Eichung bestimmt werden, beispielsweise durch Vergleich mit in herkömmlicher Weise invasiv bestimmten Blutglukosewerten.

Gemäß der Erfindung sind die optische Messeinheit, die EKG-Einheit, die Impedanzmesseinheit und ggf. der Temperatur- oder Wärmesensor in einem gemeinsamen Sensorgehäuse untergebracht. Sinnvollerweise sind die wenigstens eine EKG-Elektrode und die wenigstens eine Einspeise- oder Messelektrode der Impedanzmesseinheit als flächige Folie oder Blech aus elektrisch leitendem Material an der Oberseite des Sensorgehäuses ausgebildet. Dabei kann das Blech bzw. die Folie wenigstens eine Ausnehmung für den Durchtritt der von der wenigstens einen Strahlungsquelle emittierten Strahlung in das zu untersuchende Körpergewebe oder für den Durchtritt der von dem Körpergewebe gestreuten und/oder transmittierten Strahlung in den Strahlungssensor aufweisen. Eine weitere Ausnehmung kann für den Temperatur- oder Wärmesensor vorgesehen sein. Die Strahlungsquelle, der Strahlungssensor und der Temperatur- oder Wärmesensor können auf einer gemeinsamen Platine innerhalb des Sensorgehäuses angeordnet sein. Somit sind die benötigten Messmodalitäten in dem Sensorgehäuse zusammengefasst, welches eine Einheit bildet, die in ein beliebiges Diagnosegerät problemlos und flexibel integrierbar ist. Das Sensorgehäuse kann Abmessungen von weniger als 1 cm x 1 cm x 1 cm haben, um problemlos und flexibel im Sinne der Erfindung eingesetzt werden zu können. Dabei ist es sinnvoll, wenigstens eine EKG-Elektrode gleichzeitig auch als Einspeise- oder Messelektrode für die Bioimpedanzmessung zu benutzen. Ingesamt ergibt sich eine äußerst kompakte integrierte Messvorrichtung, die verschiedene Messmodalitäten enthält. Von sämtlichen Messmodalitäten kann derselbe Bereich des zu untersuchenden Körpergewebes (z.B. eine die Oberfläche des Sensorgehäuses berührende Fingerspitze eines Patienten) erfasst werden, um, wie oben erläutert, den Metabolismus und das Herz-/Kreislaufsystem des Patienten gleichzeitig zu untersuchen. Dies gestaltet die Durchführung einer Messung äußerst einfach und effektiv.

Gemäß einer bevorzugten Ausgestaltung der Erfindung ist die bioelektrische Impedanzmesseinheit zur Erfassung eines Impedanzmesssignals von der Hautoberfläche über wenigstens ein Messelektrodenpaar eingerichtet. Dabei beträgt der Elektrodenabstand des Messelektrodenpaares weniger als einen Millimeter bis einige Zentimeter, in der Weise, dass beim Messvorgang beide Elektroden des Messelektrodenpaares zur lokalen Erfassung des Impedanzmesssignals gleichzeitig im selben Bereich die Hautoberfläche des untersuchten Patienten berühren. Durch die Verkürzung des Elektrodenabstandes auf weniger als einen Millimeter bis zu einigen Zentimetern wird nicht, wie bei herkömmlichen Bioimpedanz-Messverfahren, über den gesamten Körper integriert, sondern die bioelektrische Impedanz wird lokal aufgenommen. Gemäß der Erfindung berühren sämtliche Elektroden ein und den selben lokalen Bereich der Hautoberfläche, d.h., dass sämtliche Elektroden mit dem selben Körperteil (z.B. Hand, Finger, Fuß oder Zehe) von dem zu untersuchenden Patienten berührt werden.

Zweckmäßig weist die erfindungsgemäße Messvorrichtung zur Messung der lokalen Resistanz und Reaktanz ein Einspeiseelektrodenpaar zur Aufprägung eines Wechselstroms variabler Frequenz über die Hautoberfläche in das Körpergewebe des zu untersuchenden Patienten auf, und zwar in dem die Messelektroden berührenden Bereich der Hautoberfläche.

Vorzugsweise beträgt der Abstand der Einspeiseelektroden, entsprechend den Messelektroden wenige Millimeter bis einige Zentimeter. Als besonders vorteilhaft hat sich eine Ausgestaltung erwiesen, bei welcher die Mess- und Einspeiseelektroden als parallel zueinander verlaufende Kontaktstreifen ausgebildet sind. Dies ermöglicht es, die lokale Impedanz des Körpergewebes ohne verfälschende Einflüsse, z.B. bedingt durch die Übergangswiderstände zwischen Elektroden und Hautoberfläche, zu bestimmen.

Zur Erzeugung des Wechselstrom variabler Frequenz weist die erfindungsgemäße Messvorrichtung zweckmäßig einen Wechselstromgenerator auf. Das Impedanzmesssignal wird durch einen Analog-/Digitalwandler digitalisiert und danach einer diskreten Fouriertransformation (DFT) unterzogen. Der DFT-Algorithmus liefert dann den Real- und den Imaginärteil der Impedanz, d.h. den Resistanz- und den Reaktanzwert. Diese Werte können zur Auswertung digital weiter verarbeitet werden.

Bevorzugt beträgt der Elektrodenabstand bis zu maximal 10 cm, besonders bevorzugt, 50 Mikrometer bis 5 cm, weiter bevorzugt 100 Mikrometer bis 1 cm, höchst bevorzugt 1 mm bis 5 mm.

Durch die Ausgestaltung der erfindungsgemäßen Messvorrichtung ist es möglich, lokale zeitliche Änderungen der Impedanz zu bestimmen. Hierzu weist die Messvorrichtung zweckmäßig eine mit der Impedanzmesseinheit verbundene Auswertungseinheit auf. Die Auswertungseinheit kann programmgesteuert sein, so dass die Auswertung der Impedanzmesssignale flexibel durch Software realisiert werden kann.

Z.B. ändert sich die lokale Bioimpedanz aufgrund der sich ändernden Blutmenge innerhalb eines Pulsschlages, wodurch eine Bestimmung der Herzfrequenz über die lokale bioelektrische Impedanz möglich ist. Als wichtiger physiologischer Parameter wird dabei gleichzeitig die Pulsamplitude bestimmt. Es hat sich gezeigt, dass diese Pulsamplitude mit der Körpertemperatur korreliert, d.h. es ist möglich, mit Hilfe der Bioimpedanzanalyse die Temperatur der untersuchten Körperstelle zu ermitteln. Weiterhin hängt die lokale Bioimpedanz von der Menge der Flüssigkeit, d.h. von der lokalen Blutmenge im untersuchten Gewebe ab, womit es möglich ist, die lokale Durchblutung (die durchblutungsbedingte lokale Volumenschwankung, z.B. in Form eines Volumenpulssignals) des untersuchten Gewebes zu bestimmen. Schließlich ändert sich die lokale bioelektrische Impedanz des Körpers in Abhängigkeit von der Nahrungsaufnahme, so dass mit der Bioimpedanz der Metabolismus untersucht werden kann, welcher bekanntlich vom Blutglukosespiegel bestimmt ist. Die erfindungsgemäße Messvorrichtung ermöglicht somit auch über die Impedanzmesseinheit eine nicht-invasive Überwachung des Blutglukosewertes, wobei die Wirkung der Glukose bzw. der Energiebedarf der durch Glukose initiierten physiologischen Reaktionen im Körper untersucht werden. Durch einen geeigneten Algorithmus, der in der Auswertungseinheit durch Software implementiert ist, ist es möglich, aus den aufgenommen Impedanzmesssignalen Aussagen über den Blutglukosespiegel und dessen zeitlichen Verlauf zu tätigen.

Gemäß einer weiteren bevorzugten Ausgestaltung der erfindungsgemäßen Messvorrichtung ist eine Fixiervorrichtung zur Fixierung eines Körperteils, z.B. eines Fingers, des zu untersuchenden Patienten vorgesehen. Bei Impedanzmessungen und auch bei pulsoximetrischen Messungen hat der Anpressdruck des Körpergewebes (z.B. des Fingers) auf den optischen Sensor bzw. auf die Mess- und Einspeiseelektroden der Impedanzmesseinheit signifikanten Einfluss auf die Messsignale. Demzufolge kann es sinnvoll sein, mittels der Fixiervorrichtung für einen definierten Anpressdruck zu sorgen. Die Fixiervorrichtung kann z.B. ein aufblasbares Luftkissen umfassen, welches das entsprechende Körperteil (sanft) gegen die Mess- und/oder Einspeiseelektroden bzw. gegen die optischen Sensoren presst und dort fixiert. Durch die Fixierung werden vorteilhaft auch Bewegungen des Körperteils unterbunden, die das Messergebnis verfälschen könnten. Die Fixiervorrichtung kann auch eine Fingerklemme an sich üblicher Art sein.

Bei einer weiteren vorteilhaften Ausgestaltung der erfindungsgemäßen Messvorrichtung ist eine Mehrzahl von Einspeise- und/oder Messelektroden matrixförmig angeordnet. Dies ermöglicht es, unterschiedliche räumliche Konfigurationen bei der Wechselstromeinspeisung und bei der Spannungsmessung zu erzeugen. Die dabei gewonnenen zusätzlichen Informationen ermöglichen es, Rückschlüsse auf den pH-Wert, den $pCO_2$-Wert, den $pO_2$-Wert sowie auf den Elektrolythaushalt ($Na^+$-, $K^+$-, $Ca^{2+}$-, $Mg^{2++}$-Konzentration etc.) zu ziehen.

Die optische Messeinheit der erfindungsgemäßen Messvorrichtung weist, wie oben ausgeführt, eine Strahlungsquelle zur Bestrahlung des untersuchten Körpergewebes mit elektromagnetischer Strahlung, und wenigstens einen Strahlungssensor zur Detektion der von dem Körpergewebe gestreuten und/oder transmittierten Strahlung auf. Als Strahlungsquelle kommen übliche Leuchtdioden oder auch Laserdioden in Frage, die optische Strahlung, d.h. Licht im entsprechenden Spektralbereich emittieren. Als besonders vorteilhaft hat es sich erwiesen, wenn mit der erfindungsgemäßen Vorrichtung die Strahlungsabsorption im untersuchten Körpergewebe bei mindestens zwei oder besser drei unterschiedlichen Lichtwellenlängen gemessen wird, um daraus die Sauerstoffkonzentration des Blutes und die Durchblutung des Gewebes zu bestimmen.

Gemäß einer sinnvollen Ausgestaltung weist die optische Messeinheit der erfindungsgemäßen Messvorrichtung wenigstens zwei Strahlungssensoren zur Detektion der von dem Körpergewebe gestreuten und/oder transmittierten Strahlung auf, wobei die Strahlungssensoren in unterschiedlichem Abstand zur Strahlungsquelle angeordnet sind. Dies eröffnet die Möglichkeit, Rückschlüsse auf die jeweils im Körpergewebe von der Strahlung zurückgelegte Strecke zu ziehen. Auf dieser Basis kann die Sauerstoffkonzentration im Blut und im Gewebe in unterschiedlich tiefen Gewebeschichten untersucht werden. Dabei kann ausgenutzt werden, dass die Messsignale aus den tiefer liegenden Gewebeschichten stärker vom arteriellen Blut beeinflusst sind, während in den oberflächennäheren Regionen die Strahlungsabsorption stärker von dem Blut im kapillaren Gefäßsystem beeinflusst ist. Sinnvoll ist es auch, einen Strahlungssensor zur Detektion von transmittierter Strahlung und einen weiteren Strahlungssensor zur Detektion von am Körpergewebe (zurück) gestreuter Strahlung zu verwenden. Die in Transmission erfassten Messsignale sind stärker von der Absorption im arteriellen Blut beeinflusst, während die gestreute Strahlung hauptsächlich aus oberflächennahem Gewebe stammt und daher Rückschlüsse auf die Absorption und damit den Sauerstoffgehalt im kapillaren Gefäßsystem erlaubt.

Vorteilhaft ist eine Ausgestaltung der erfindungsgemäßen Messvorrichtung, bei welcher die Abstrahlung des Lichtes der wenigstens einen Strahlungsquelle in der Weise erfolgt, dass verschiedene Volumenbereiche des untersuchten Körpergewebes selektiv bestrahlt werden. Hierzu können z.B. zwei Strahlungsquellen vorgesehen sein, welche die unterschiedlichen Volumenbereiche des untersuchten Körpergewebes bestrahlen. Hierdurch lässt sich eine differenzielle Messung der Lichtabsorption einfach realisieren. Dies ermöglicht es, Metabolismus-induzierte Änderungen der Durchblutung des untersuchten Körpergewebes mit sauerstoffreichem bzw. sauerstoffarmem Blut zu untersuchen. Dabei wird ausgenutzt, dass sich in Abhängigkeit von der metabolischen Aktivität des Gewebes der lokale Sauerstoffverbrauch verändert. Die Bestimmung des veränderlichen Sauerstoffverbrauchs erlaubt wiederum Rückschlüsse auf den lokalen Energieverbrauch, der mit dem Sauerstoffverbrauch direkt korreliert ist. Besonders interessant ist, dass dies wiederum Rückschlüsse auf den Glukosespiegel zulässt. Somit erlaubt die erfindungsgemäße Messvorrichtung vorteilhafterweise auch eine nicht-invasive Bestimmung des Blutglukosespiegels. Diese jeweils selektiv bestrahlten Volumenbereiche sollten hinsichtlich der Durchblutung mit sauerstoffarmem bzw. sauerstoffreichem Blut unterschiedlich betroffen sein. Dies kann z. B. dadurch erreicht werden, dass die wenigstens zwei Strahlungsquellen unterschiedliche räumliche Abstrahlcharakteristiken haben. So können als Strahlungsquellen z. B. eine Leuchtdiode und ein Laser verwendet werden, die ähnliche Wellenlängen (z. B. 630 nm und 650 nm) haben. Die beiden Strahlungsquellen unterscheiden sich aber durch den Öffnungswinkel der Abstrahlung. Während z. B. die Leuchtdiode unter einem großen Öffnungswinkel in das untersuchte Körpergewebe einstrahlt, tritt das Licht der Laserdiode unter einem sehr kleinen Öffnungswinkel in das Körpergewebe ein. Dies hat zur Folge, dass mit den beiden Strahlungsquellen unterschiedliche Volumenbereiche des Körpergewebes erfasst werden. Aufgrund des großen Öffnungswinkels wird von der Leuchtdiode ein größerer Volumenbereich der nicht-durchbluteten Epidermis erfasst als von dem Laser. Die undurchblutete Epidermis ist von einer Änderung der Hämoglobinkonzentration praktisch nicht betroffen. Dementsprechend ist die Intensität der von dem Körpergewebe gestreuten und/oder transmittierten Strahlung der Leuchtdiode weniger stark von einer Änderung der Hämoglobinkonzentration abhängig als die Intensität der Strahlung des Lasers. Voraussetzung ist, dass die Wellenlänge der von den beiden Strahlungsquellen jeweils emittierten Strahlung so gewählt wird, dass die Strahlung unterschiedlich stark durch Oxihämoglobin bzw. Desoxihämoglobin absorbiert wird. Die Wellenlänge sollte daher zwischen 600 und 700 nm, vorzugsweise zwischen 630 und 650 nm liegen.

Die erfindungsgemäße Messvorrichtung kann mit Vorteil zur Bestimmung eines metabolischen Parameters aus der von dem Körpergewebe gestreuten und/oder transmittierten Strahlung der wenigstens einen Strahlungsquelle ausgebildet sein. Wenn in dem untersuchten Körpergewebe Sauerstoff verbraucht wird, wird Oxihämoglobin in Desoxihämoglobin umgewandelt. Durch einen Vergleich der aus den selektiv bestrahlten unterschiedlichen Volumenbereichen des Körpergewebes stammenden Strahlung kann die Änderung des Konzentrationsverhältnisses von Oxihämoglobin und Desoxihämoglobin festgestellt werden. Hieraus ergibt sich wiederum der lokale Sauerstoffverbrauch und daraus letztlich (indirekt) der Blutglukosespiegel.

An den gemäß der Erfindung vorgesehenen Hohlreflektor kann eine Optik angebunden werden, die eine gerichtete Abstrahlung in das Körpergewebe bewirkt. Weiterhin kann der Hohlreflektor eine Austrittsöffnung aufweisen, durch welche eine diffuse Abstrahlung in das Körpergewebe erfolgt. Auf diese Weise kann die oben beschriebene optische Messung an unterschiedlichen Volumenbereichen des Körpergewebes erfolgen.

Wenn der Hohlreflektor, wie oben beschrieben, ein Chipgehäuse ist, kann vorteilhaft die Optik durch ein das Chipgehäuse ausfüllendes transparentes Kunststoffmaterial (z.B. Silikonharz oder Epoxidharz) gebildet sein. Dies ermöglicht einen sehr kompakten und robusten Aufbau.

Für die praktische Nutzung kann die erfindungsgemäße Messvorrichtung an ein beliebiges programmgesteuertes Gerät, beispielsweise einen Computer, ein Mobiltelefon, ein Handheld, etc. angeschlossen werden, wobei die Funktionen zur Auswertung der erfassten Messsignale durch auf dem programmgesteuerten Gerät ablaufende Software realisiert sind. Aufgrund der geringen Größe des Sensorgehäuses kann dieses auch in ein beliebiges Accessoire, wie z.B. eine Brille, eine Armbanduhr, ein Schmuckstück oder dergleichen, oder in ein Kleidungsstück (sog. "Smart Clothes") integriert werden. Bei dieser Ausgestaltung wird z.B. die bei dem programmgesteuerten Gerät ohnehin vorhandene Datenverarbeitungselektronik zur Verarbeitung der gewonnenen Messsignale benutzt. Dies lässt sich durch Bereitstellung entsprechender Software leicht bewerkstelligen. Gleichzeitig können die mittels der Software ermittelten diagnostischen Daten gespeichert werden. Dies ermöglicht es, den Verlauf einer Erkrankung und die Effekte einer entsprechenden Therapie zu verfolgen und zu dokumentieren. Sinnvollerweise kann auch eine Datenfernübertragung der mittels der Messvorrichtung erfassten und ausgewerteten diagnostischen Daten erfolgen. Die Datenübertragung kann z.B. über ein Datennetz (z.B. Internet) erfolgen. Alternativ können die diagnostischen Daten über ein Mobilfunknetz übermittelt werden, wenn die Messvorrichtung gemäß der Erfindung z.B. in ein Mobiltelefon integriert ist. Die rohen Messsignale oder die ausgewerteten diagnostischen Daten können zum Beispiel an eine zentrale Stelle ("Healthcare-Center") zur eingehenderen Analyse und Dokumentation sowie zur Überwachung der zeitlichen Entwicklung einzelner Werte übermittelt werden. Dort werden die Daten z.B. mittels geeigneter Analysealgorithmen ggf. unter Berücksichtigung von hinterlegten Patientendaten (einschließlich Informationen bezüglich chronischer Erkrankungen oder Vorerkrankungen) ausgewertet. Das Ergebnis kann wiederum über das jeweilige Daten- oder Kommunikationsnetz z.B. an das Mobiltelefon zurück gesendet werden, um den Benutzer der Vorrichtung entsprechend über seinen Gesundheitszustand zu informieren. Von der zentralen Stelle aus können auch bei Bedarf weitere gezielte Messungen mittels der erfindungsgemäßen Messvorrichtung initiiert werden. Außerdem können zum Zwecke einer erweiterten Anamnese veranlasst durch die Auswertungsergebnisse Rückfragen an den Patienten über das Daten- oder Kommunikationsnetz übermittelt werden. Die Daten und Auswertungsergebnisse können automatisch an einen behandelnden Arzt übermittelt werden. Falls sich aus den Mess- und Auswertungsergebnissen Hinweise auf einen medizinischen Notfall ergeben, können die erforderlichen Maßnahmen (z.B. automatische Alarmierung des Rettungsdienstes) sofort in die Wege geleitet werden. Ein weiterer Vorteil der Datenfernübertragung ist, dass die erforderliche Software zur Auswertung der Messsignale nicht in der Vorrichtung selbst implementiert sein muss, sondern lediglich an der zentralen Stelle, wo die Daten empfangen werden, vorgehalten und gepflegt werden muss.

Bei pulsoximetrischen Messungen hat der Anpressdruck des Körpergewebes (z.B. des Fingers) auf den optischen Sensor signifikanten Einfluss auf die Messsignale. Demzufolge kann es sinnvoll sein, die erfindungsgemäße Messvorrichtung mit Mitteln zur Bestimmung des Anpressdrucks des Körpergewebes auszustatten. Es kann sich dabei um einen herkömmlichen Drucksensor, beispielsweise in Form eines piezoresistiven Elements handeln. Ebenso möglich sind optische Verfahren zur Bestimmung des Anpressdrucks. Denkbar ist es auch, den Anpressdruck aus den (pulsoximetrischen) Signalen selbst zu bestimmen, da sich der Anpressdruck charakteristisch auf die Messsignale auswirkt. Der ermittelte Anpressdruck kann dann bei der weiteren Auswertung der Messsignale berücksichtigt werden, um den Einfluss des Anpressdrucks beispielsweise auf die Durchblutung zu kompensieren.

Ausführungsbeispiele der Erfindung werden im Folgenden unter Bezugnahme auf die Zeichnungen näher erläutert. Es zeigen:

Figur 1     Schematische Ansicht der Integration der erfindungsgemäßen Messvorrichtung in eine Computertastatur;

Figur 2     Darstellung der Funktion der erfindungsgemäßen Messvorrichtung anhand eines Blockdiagramms;

Figur 3     weiteres Ausführungsbeispiel der erfindungsgemäßen Messvorrichtung;

Figur 4     Hohlreflektor mit Strahlungsquelle;

Figur 5     Ausführungsbeispiel einer Konfiguration der optischen Messeinheit der erfindungsgemäßen Messvorrichtung;

Figur 6     Gehäuseoberfläche des Sensorgehäuses mit Elektroden, Strahlungsquelle, Strahlungssensoren und Wärmesensor in einer ersten möglichen Konfiguration;

Figur 7     Gehäuseoberfläche des Sensorgehäuses in einer zweiten möglichen Konfiguration;

Figur 8     Konfiguration der Gegenelektroden zur EKG- und Bioimpedanzmessung;

Figur 9     weiteres Ausführungsbeispiel der erfindungsgemäßen Messvorrichtung;

Figur 10 Chipgehäuse als Hohlreflektor in Draufsicht;

Figur 11 Schnittdarstellung des Chipgehäuses gemäß Figur 10.

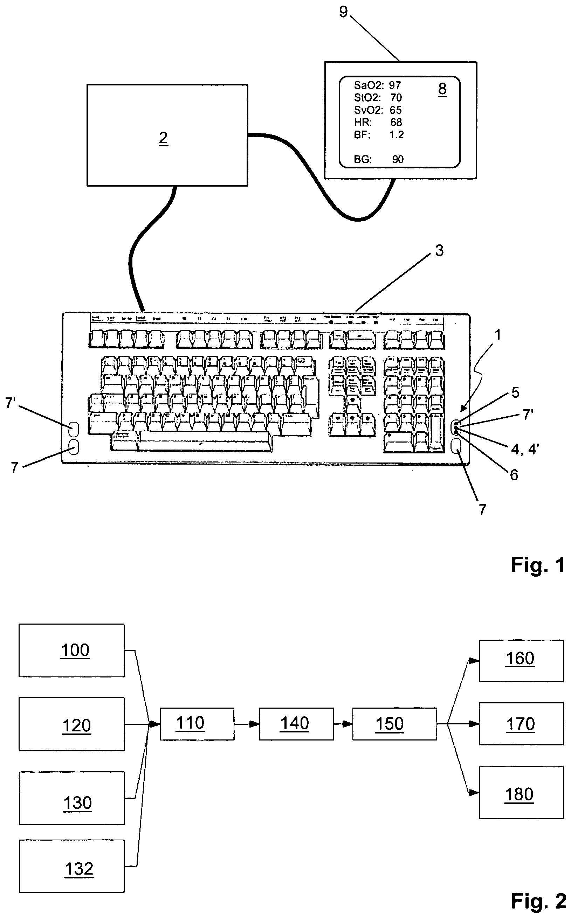

Die Figur 1 zeigt eine insgesamt mit der Bezugsziffer 1 bezeichnete Messvorrichtung gemäß der Erfindung, die in ein Computersystem bestehend aus Computer 2 und Tastatur 3 integriert ist. Die Messvorrichtung 1 weist verschiedene Messmodalitäten auf, die an der Bedienoberfläche der Tastatur 3 zugänglich sind. Diese berührt der Benutzer des Computersystems zur Durchführung einer Messung mit den Fingerspitzen. In die Messvorrichtung 1 sind Lichtquellen 4, 4' beispielsweise in Form von Leuchtdioden integriert, die dazu in der Lage sind, Licht bei verschiedenen Wellenlängen zu emittieren. Hierzu sind verschiedene lichtemittierende Halbleiterelemente in einem gemeinsamen Sensorgehäuse (in Figur 1 das Gehäuse der Tastatur) untergebracht. Ebenso denkbar ist die Verwendung von Lichtwellenleitern, um das Licht von verschiedenen Lichtquellen an die Bedienoberfläche der Tastatur 3 zu führen (s.u.). Des Weiteren umfasst die Messvorrichtung 1 einen oder mehrere Fotosensoren 5. Die Fotosensoren sind in unmittelbarer Nähe zur Lichtquelle 4 bzw. 4' angeordnet. Die Sensoren 5 empfangen das im Gewebe an der Fingerspitze des Benutzers gestreute Licht der Lichtquelle 4 bzw. 4'. Weiterhin ist unmittelbar neben der Lichtquelle 4 bzw. 4' ein Wärmesensor 6 vorgesehen. Dadurch ist gewährleistet, dass die Bestimmung der Durchblutung anhand der Wärmemessung am selben Messort erfolgt wie die optische Messung. Außerdem sind an der Oberfläche des Sensorgehäuses insgesamt vier Elektroden 7 bzw. 7' zur Messung der bioelektrischen Impedanz vorgesehen. Der Benutzer der Vorrichtung berührt mit einer Hand jeweils zwei Elektroden 7 bzw. 7' gleichzeitig. Eine der beiden Kontaktflächen dient zur Aufprägung eines elektrischen Stromes am Messort, während die andere Kontaktfläche zur Spannungsmessung genutzt wird. Auf diese Weise ist sichergestellt, dass die Messergebnisse nicht von den Kontaktwiderständen der Messelektroden beeinflusst sind. Die beiden mit der Bezugsziffer 7 bezeichneten Elektroden werden außerdem als EKG-Elektroden einer ebenfalls in die Messvorrichtung 1 integrierten EKG-Einheit verwendet. Die beiden Elektroden werden jeweils mit den Fingerspitzen berührt, so dass sich eine Zweipunkt-Ableitung (Arm-zu-Arm-Messung) ergibt. Die mittels der in die Tastatur 3 integrierten Messvorrichtung 1 aufgenommenen Messsignale werden mittels des Computers 2 verarbeitet. Die so gewonnenen physiologischen Parameter werden dann auf einer Anzeigefläche 8 eines an den Computer 2 angeschlossenen Monitors 9 ausgegeben. Angezeigt werden z. B. die arterielle ($SaO_2$), die kapillare ($StO_2$) und die venöse ($SvO_2$) Sauerstoffsättigung. Angezeigt wird weiterhin die ermittelte Herzfrequenz (HR), der Fettgehalt des Gewebes (BF).

Schließlich wird noch ein Blutglucosewert (BG) angezeigt. Der Benutzer kann jederzeit die ihn interessierenden physiologischen Parameter ermitteln. Hierzu legt er lediglich die Finger, mit denen er ansonsten die Tasten der Tastatur 3 betätigt, auf die Elektroden 7, 7'. Die Parameter werden dann nach der Verarbeitung der Messsignale mittels des Computers 2 sofort mittels des Monitors 9 angezeigt. Der Benutzer der Vorrichtung 1 muss seine Arbeit am Computer 2 also zur Ermittlung der physiologischen Parameter praktisch nicht unterbrechen.

Bei dem in der Figur 1 dargestellten Ausführungsbeispiel der Messvorrichtung 1 sind zwei Strahlungsquellen 4 und 4' vorgesehen, welche unterschiedliche Volumenbereiche des untersuchten Körpergewebes bestrahlen. Hierzu haben die zwei Strahlungsquellen 4 und 4' unterschiedliche räumliche Abstrahlcharakteristiken, nämlich unterschiedliche Abstrahlwinkel. Bei der Strahlungsquelle 4 handelt es sich um eine Leuchtdiode, während es sich bei der Strahlungsquelle 4' um einen Laser, beispielsweise einen so genannten VCSEL-Laser (engl. "vertical cavity surface emitting laser") handelt. Sowohl die Leuchtdiode 4 als auch der Laser 4' emittieren Licht mit sehr ähnlicher Wellenlänge (z. B. 630 nm und 650 nm), aber mit unterschiedlichen Öffnungswinkeln (z. B. 25° und 55°). Mit der in der Figur 1 dargestellten Anordnung ist - wie oben beschrieben - eine differenzielle Messung von Metabolismus-induzierten Änderungen des Sauerstoffgehalts im Blut möglich. Hierzu muss die Wellenlänge der von den beiden Strahlungsquellen 4 und 4' jeweils emittierten Strahlung in einem Bereich liegen, in welchem das Licht von Oxihämoglobin und Desoxihämoglobin unterschiedlich stark absorbiert wird. Für eine Absolutmessung des Sauerstoffgehalts des Blutes (Sauerstoffsättigung) müssen weitere Strahlungsquellen (in der Figur 1 nicht dargestellt) vorhanden sein, deren Lichtwellenlänge in einem Spektralbereich liegt, in welchem die Lichtabsorption von Oxihämoglobin und Desoxihämoglobin im Wesentlichen gleich ist (so genannter isobestischer Punkt). Das von der Leuchtdiode bzw. von dem Laser emittierte Licht kann mittels entsprechender Lichtleitfasern an die entsprechende Stelle an der Bedienoberfläche der Tastatur geführt werden. In diesem Fall sind mit den Bezugsziffern 4 und 4' in der Figur 1 die entsprechenden Faserenden dargestellt. Es ist möglich, die Leuchtdiode und den Laser so an die entsprechenden Fasern anzukoppeln, dass sie mit dem gewünschten unterschiedlichen Öffnungswinkel in das zu untersuchende Körpergewebe einstrahlen. Dementsprechend werden mit beiden Strahlungsquellen unterschiedliche Volumina des Körpergewebes untersucht. Aufgrund des größeren Öffnungswinkels ist der Anteil der nicht-durchbluteten Epidermis an dem mittels der Leuchtdiode untersuchten Körpergewebe größer als beim Laser. Das im Körpergewebe gestreute und teilweise absorbierte Licht sowohl der Strahlungsquelle 4 als auch der Strahlungsquelle 4' wird mittels der Sensoren 5 detektiert. Die Sensoren 5 müssen nicht direkt an der Oberfläche der Messvorrichtung 1 angeordnet sein. Stattdessen kann das Licht über Lichtleitfasern den im Inneren der Messvorrichtung 1 angeordneten Sensoren zugeführt werden. Zur Unterscheidung des Lichtes der Strahlungsquelle 4 von dem Licht der Strahlungsquelle 4' können die beiden Lichtquellen 4 und 4' unterschiedlich zeitlich moduliert betrieben werden, wobei die mittels der Sensoren 5 detektierten Signale entsprechend demoduliert werden. Alternativ ist es möglich, die Strahlung der beiden Strahlungsquellen 4 und 4' anhand der unterschiedlichen Wellenlänge zu unterscheiden. Die Strahlungsintensität der von den Strahlungsquellen 4 und 4' emittierten Strahlung wird mit der Weglänge beim Durchgang durch das Körpergewebe geschwächt, wobei der Zusammenhang der Intensitätsschwächung mit der Konzentration der absorbierenden Substanz (oxigeniertes Hämoglobin) durch das bekannte Lambert-Beersche Gesetz gegeben ist. Mittels der in der Figur 1 dargestellten Sensoren 5 können die interessierenden Parameter der Intensitätsschwächung bestimmt werden, und zwar getrennt für die von den Strahlungsquellen 4 und 4' jeweils erfassten Volumenbereiche des untersuchten Körpergewebes. Die den verschiedenen Strahlungsquellen 4 und 4' zuzuordnenden Parameter der Intensitätsschwächung können mittels einer geeignet programmgesteuerten Auswertungseinheit zueinander in Beziehung gesetzt werden, um auf diese Weise eine differenzielle Messung durchzuführen. Im einfachsten Fall werden aus den Parametern der Intensitätsschwächung der Strahlung der beiden Strahlungsquellen 4 und 4' jeweils Quotienten berechnet. Aus Änderungen dieser Quotienten kann dann auf Änderungen im Metabolismus zurückgeschlossen werden. Steigt beispielsweise nach der Nahrungsaufnahme der Blutglukosespiegel, gelangt (nach einer gewissen zeitlichen Verzögerung) entsprechend mehr Glukose in die Zellen des Körpergewebes und wird dort umgesetzt. Dabei wird Sauerstoff verbraucht. Diesen Sauerstoff erhalten die Zellen über das Blut. Dabei wird aus dem oxigenierten Hämoglobin durch Abgabe von Sauerstoff desoxigeniertes Hämoglobin. Dementsprechend steigt das Verhältnis von desoxigeniertem Hämoglobin zu oxigeniertem an. Aufgrund der unterschiedlichen Öffnungswinkel der Strahlung der Strahlungsquellen 4 und 4' wirken sich die Änderungen der Hämoglobinkonzentration unterschiedlich auf die jeweilige Intensitätsschwächung aus. Somit können aus den Quotienten der Parameter der Intensitätsschwächung Veränderungen der Hämoglobinkonzentration detektiert werden. Dies ermöglicht es, indirekt auf den Sauerstoffverbrauch zurückzuschließen. Da der Sauerstoffverbrauch seinerseits vom Blutglukosespiegel abhängt, kann mittels der erläuterten differenziellen Messung der Strahlungsabsorption auch der Blutglukosespiegel ermittelt werden. Als Ergänzung wird parallel zur optischen Messung eine Bioimpedanzanalyse durchgeführt, wozu die in der Figur 1 dargestellten Elektroden 7 und 7' vorgesehen sind. Zweck der Bioimpedanzmessung ist vor allem die Bestimmung der lokalen Durchblutung. Diese kann als weiterer Parameter bei der Bestimmung des Sauerstoffverbrauchs und damit auch des Blutglukosespiegels herangezogen werden. Unterschiedliche Öffnungswinkel der Strahlung können auch mit nur einer Strahlungsquellen 4 durch Verwendung entsprechender optischer Elemente (z.B. Strahlteiler, Linsen etc.) erzeugt werden.

Weiterhin vorteilhaft ist es, die Auswertung der Messwerte Puls-synchron durchzuführen. Dies ist möglich, da die erfindungsgemäße Messvorrichtung über geeignete Sensorik zur Pulserfassung (z.B. EKG) verfügt. So können z.B. die mittels optischer Messung oder Impedanzmessung gewonnenen Messwerte gezielt zum Zeitpunkt des Maximums und/oder des Minimums der Pulswelle ausgewertet werden, um pulsbedingte Schwankungen auszugleichen. Auch können aus dem Verhältnis der pulsabhängigen zur pulsunabhängigen Blutmenge im untersuchten Körpergewebe sowie aus den pulsabhängigen und pulsunabhängigen metabolischen Daten, die mit der erfindungsgemäßen Vorrichtung separat erfassbar sind, wertvolle diagnostische Informationen gewonnen werden.

Die Figur 2 zeigt schematisch den Aufbau der erfindungsgemäßen Messvorrichtung 1 als Blockdiagramm. Die Messvorrichtung 1 umfasst eine optische Messeinheit 100 zur optischen Messung der Sauerstoffkonzentration im Blutgefäßsystem des Körpergewebes am jeweiligen Messort. Die mittels der optischen Messeinheit 100 erfassten oximetrischen und plethysmographische Signale werden einer Analyseeinheit 110 zugeführt. Eine weitere wesentliche Komponente der Vorrichtung 1 ist eine Wärmemesseinheit 120 zur Bestimmung der lokalen Wärmeproduktion. Bei der Wärmemesseinheit 120 handelt es sich um einen speziellen Wärmesensor, welcher die jeweils untersuchte Körperstelle isoliert. Diese Stelle kann somit nur noch Wärme durch den Blutstrom aufnehmen oder abgeben. Daher ist es möglich, durch die zeitaufgelöste Messung der Temperatur die Durchblutung und die Wärmeproduktion zu bestimmen. Bei einer starken Durchblutung erreicht die untersuchte Körperstelle in sehr kurzer Zeit ihre maximale Temperatur. Bei geringer Durchblutung dauert dies länger. Zusätzlich kann über die Extrapolation der gemessenen Temperatur auf die arterielle Temperatur geschlossen werden, da die Temperatur am Ort der Messung nur durch die arterielle Temperatur und durch die lokale Wärmeproduktion bestimmt wird. Auch die mittels der Wärmemesseinheit 120 erfassten Messsignale werden der Analyseeinheit 110 zur Weiterverarbeitung zugeführt. Außerdem umfasst die Vorrichtung eine Impedanzmesseinheit 130, die zur Erfassung von lokalen Gewebeparametern mittels bioelektrischer Impedanzmessung dient. Die Messsignale der Impedanzmesseinheit 130 werden ebenfalls mittels der Analyseeinheit 110 verarbeitet. Schließlich ist gemäß der Erfindung noch eine EKG-Einheit 132 zur Erfassung eines EKG-Signals vorgesehen. Auch die EKG-Einheit 132 ist zur Verarbeitung der EKG-Signale mit der Analyseeinheit 110 verbunden. Der optischen Messeinheit 100 sind die Lichtquelle 4 sowie die Lichtsensoren 5 der in der Figur 1 dargestellten Messvorrichtung 1 zugeordnet. Die Wärmemesseinheit 120 ist mit dem Wärmesensor 6 verbunden. Die Impedanzmesseinheit 130 erfasst Messsignale über die Elektroden 7 bzw. 7' der Vorrichtung 1. Die Analyseeinheit 110 führt eine Vorverarbeitung sämtlicher Messsignale durch. Hierzu durchlaufen die Signale ein Bandpass-Filter, um Störungen im Bereich der Netzfrequenz von 50 bzw. 60 Hz herauszufiltern. Des Weiteren werden die Signale einer Rauschunterdrückung unterzogen. Nach Passieren der Analyseeinheit 110 gelangen die aufbereiteten Signale der optischen Messeinheit 100, der Wärmemesseinheit 120, der Impedanz-Messeinheit 130 und der EKG-Einheit 132 in eine Auswertungseinheit 140. Die Auswertungseinheit 140 ist dafür zuständig, aus den Messsignalen die für die Diagnose wesentlichen Parameter zu berechnen. Aus den zeitabhängig aufgenommenen Messsignalen der Impedanzmesseinheit 130 wird zunächst die Zusammensetzung des untersuchten Körpergewebes (Wassergehalt, Fettgehalt usw.) berechnet. Aus den Signalen der optischen Messeinheit 100 wird die arterielle Sauerstoffsättigung und - unter Zugrundelegung der auf der Basis der Impedanzmessung ermittelten Gewebeparameter - die kapillare Sauerstoffsättigung berechnet. Weiterhin werden aus den Messsignalen der Wärmemesseinheit 120 und aus den plethysmographischen Daten, die aus der zeitabhängigen Impedanzmessung ableitbar sind, die Durchblutung und die arterielle Temperatur bestimmt. Aus den Signalen der EKG-Einheit 132 und denjenigen der optischen Messeinheit 100 wird die Pulswellengeschwindigkeit bestimmt. Schließlich werden mittels der Auswertungseinheit 140 aus den Ergebnissen sämtlicher zuvor durchgeführter Berechnungen die venöse Sauerstoffsättigung, und daraus weitere metabolische Parameter, insbesondere der lokale Sauerstoffverbrauch und die Glukosekonzentration am Messort berechnet. Die Berechnungsergebnisse werden mittels einer Diagnoseeinheit 150 interpretiert. Die Diagnoseeinheit 150, die ebenfalls als Software auf dem Computer 2 implementiert ist, dient zur Bewertung der mittels der Auswertungseinheit 140 berechneten lokalen metabolischen Parameter. Die Auswertungseinheit 140 und die Diagnoseeinheit 150 sind zur Anzeige der Messresultate mit einer Grafikeinheit 160 verbunden, die ihrerseits den Monitor 9 ansteuert. Die gewonnenen Daten sind in einer Speichereinheit 170 speicherbar, und zwar unter gleichzeitiger Speicherung des Datums und der Uhrzeit der jeweiligen Messung. Außerdem ist eine Schnittstelleneinheit 180 vorgesehen, die zur Verbindung des Computers 2 mit einem Datennetzwerk zur Übertragung der berechneten physiologischen Parameter dient. Über die Schnittstelleneinheit 180 können sämtliche Daten und Parameter, insbesondere auch die in der Speichereinheit 170 gespeicherten Daten und Parameter, an einen nicht näher dargestellten PC eines behandelnden Arztes übertragen werden. Dort können die Daten detaillierter analysiert werden. Insbesondere können über einen längeren Zeitraum mit der Messvorrichtung 1 aufgenommene Daten und Parameter auf Veränderungen hin untersucht werden, um daraus Schlussfolgerungen hinsichtlich der Entwicklung einer bestehenden Erkrankung ableiten zu können.

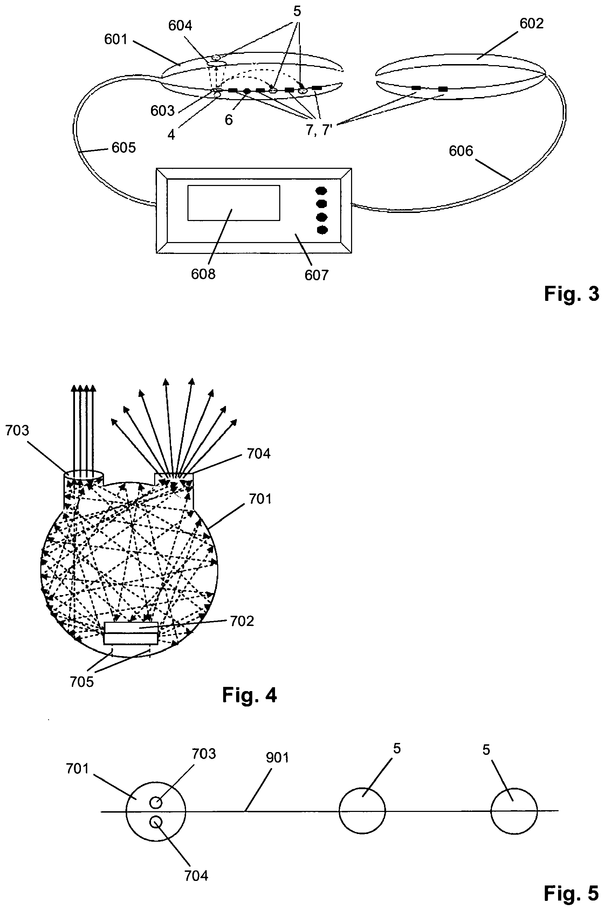

Die Figur 3 zeigt ein alternatives Ausführungsbeispiel der erfindungsgemäßen Messvorrichtung. Diese umfasst zwei Fingerklemmen 601 und 602, durch welche jeweils ein Finger der linken und rechten Hand an den Sensoren der Messvorrichtung fixiert wird. Die Strahlungsquelle 4, die Strahlungssensoren 5, die zur EKG- und Impedanzmessung verwendeten Elektroden 7, 7' sowie der Wärmesensor 6 sind in die Fingerklemme 601 integriert. Dabei ist eine Besonderheit des in der Figur 3 dargestellten Ausführungsbeispiels, dass das von der Strahlungsquelle 4 erzeugte Licht mittels der Strahlungssensoren 5 auf zweifache Weise gemessen wird, nämlich zum einen in Transmissionsrichtung mittels des Strahlungssensors 5 der in dem oberen Teil der Fingerklemme 601 der Strahlungsquelle 4 gegenüberliegend angeordnet ist, und zum anderen mittels der in den unteren Teil der Fingerklemme 601 integrierten Strahlungssensoren 5, die das im Gewebe des Fingers zurückgestreute Licht detektieren. Für die Transmissionsmessung sind Linsen 603, 604 jeweils von der Strahlungsquelle 4 und dem gegenüberliegenden Strahlungssensor 5 angeordnet. Die Linse 603 sorgt für eine gerichtete Abstrahlung des Lichtes in das Gewebe. Die Linse 604 sammelt das transmittierte Licht und fokussiert dieses auf den Strahlungssensor 5. In die Fingerklemme 602 sind lediglich zwei Elektroden 7, 7' als Gegenelektroden zu den in die Fingerklemme 601 integrierten Elektroden angeordnet. Diese dienen zur EKG-Messung (Zweipunktableitung) sowie zur (globalen) Bioimpedanzmessung. Die Fingerklemmen 601 und 602 sind über Kabel 605, 606 mit einer Zentraleinheit 607 verbunden. Die Zentraleinheit 607 enthält die Analyse-, Auswertungs- und Diagnoseeinheiten der Messvorrichtung. Die Darstellung der gewonnenen Messwerte erfolgt über das Display 608. Mit der dargestellten Anordnung sind verschiedene optische Messwerte, z.B. in Form von über die Strahlungssensoren erfassten Lichtintensitäten, messbar. Dies ist die transmittierte Intensität $I_T$ und die aus unterschiedlichen Gewebebereichen zurückgestreuten Intensitäten $I_{RS1}$ und $I_{RS2}$. Zur Kalibrierung kann eine Messung an einem (künstlichen) Standardmaterial durchgeführt werden. Dies ergibt dann den Wert $I_{ref}$. Diese vier Messwerte bilden die Grundlage für die weitere Auswertung.

Die Figur 4 zeigt einen Hohlreflektor 701, in dem als Strahlungsquelle eine (oder mehrere) LED 702 angeordnet ist. Es handelt sich um eine SMD-LED, die in alle Raumrichtungen Licht emittiert. Andere Arten von Lichtquellen sind gemäß der Erfindung ebenfalls möglich. Das Licht der LED 702 wird an den inneren Oberflächen des Hohlreflektors 701 diffus reflektiert. Der Hohlreflektor 701 ist bei dem Ausführungsbeispiel nach Art einer Ulbricht-Kugel ausgebildet. An den Hohlreflektor 701 ist eine Optik 703 in Form einer Sammellinse angebunden. Die Sammellinse bewirkt, wie in der Figur 4 angedeutet, eine gerichtete Abstrahlung des von der LED 702 abgestrahlten Lichtes. Weiterhin weist der Hohlreflektor 701 eine Austrittsöffnung 704 auf, durch welche eine diffuse Abstrahlung in das Körpergewebe erfolgt. Auch im Bereich der Austrittsöffnung 704 kann eine geeignete Optik vorgesehen sein, um den gewünschten Abstrahlwinkel zu erzielen. Unterschiedliche Abstrahlwinkel können z.B. mittels Linsen unterschiedlicher Brennweiten oder anderer optischer Elemente, wie z.B. Kollimatoren etc., erzeugt werden. Die in Figur 4 gezeigte Anordnung kann mit Vorteil als kompakte vorgefertigte Einheit zu geringen Kosten in großen Stückzahlen hergestellt werden. Die elektrischen Anschlüsse 705 der LED sind aus dem Hohlreflektor heraus geführt. Der Hohlreflektor kann z.B. als massiver Körper aus transparentem Kunststoff ausgebildet sein, in den die LED 702 eingebettet ist. Dieser kann dann an seiner Außenseite mit diffus reflektierendem Material (z.B. Aluminiumoxid oder Bariumsulfat) beschichtet sein. Entscheidend kommt es darauf an, dass durch diffuse Reflektion die Richtcharakterisitik der LED aufgehoben wird und dann, durch Auskopplung des Lichtes in geeigneter Weise aus dem Reflektor, die gewünschte Abstrahlcharakteristik nach den jeweiligen Anforderungen vorgegeben werden kann. Es ist problemlos möglich, mehrere LEDs in einem Hohlreflektor unterzubringen, um Strahlung bei verschiedenen Wellenlängen zu erzeugen und einheitlich über die an den Hohlreflektor 701 angebundene Optik 703 bzw. über die Austrittföffnung 704 in das Körpergewebe einzustrahlen.

Die Figur 5 zeigt eine Draufsicht auf die Sensorik der optischen Messeinheit an der Oberfläche des Sensorgehäuses der erfindungsgemäßen Messvorrichtung, welche an dem zu untersuchenden Körpergewebe anliegt. Zu sehen ist die Oberseite des Hohlreflektors 701, wobei die Optik 703 und die Austrittsöffnung 704 in der Ebene der Messoberfläche liegen. Dabei sind die Optik 703 und die Austrittsöffnung 704 jeweils links und rechts einer Achse 901 angeordnet. Auf der Achse 901 sind weiterhin Strahlungssensoren 5 angeordnet. Jeder der Strahlungssensoren 5 hat jeweils den gleichen Abstand von der Optik 703 und der Austrittsöffnung 704. Gleichzeitig haben die beiden Strahlungssensoren 5 unterschiedliche Abstände zur Strahlungsquelle. Diese Anordnung eröffnet die Möglichkeit, die im Körpergewebe von der detektierten Strahlung zurückgelegte Strecke zu berücksichtigen, so dass die Sauerstoffkonzentration im Blut und im Gewebe in unterschiedlich tiefen Gewebeschichten untersucht werden kann.

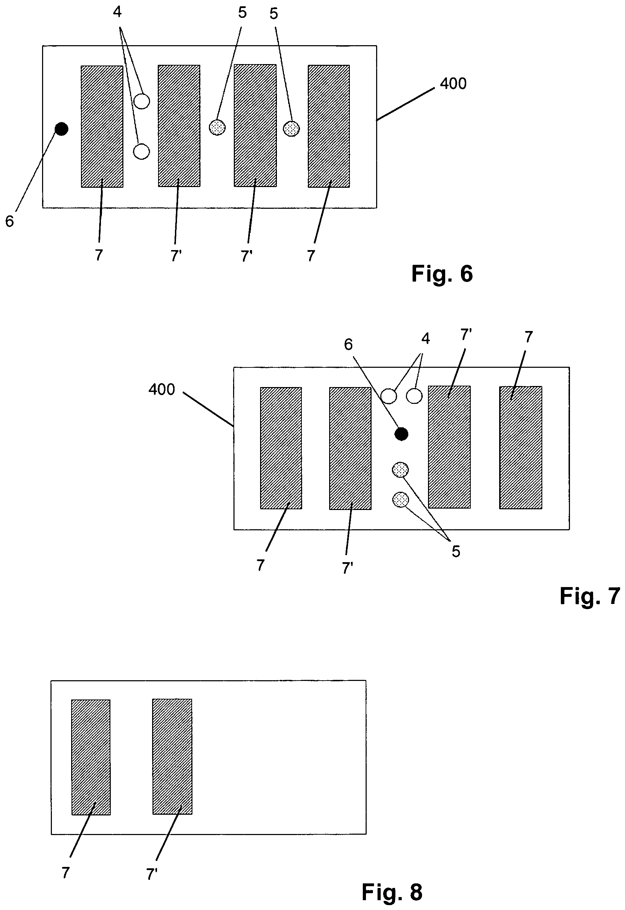

Die Figuren 6, 7 und 8 zeigen unterschiedliche Konfigurationen der Sensorik der erfindungsgemäßen diagnostischen Messvorrichtung. Die Figuren 6 und 7 zeigen zwei verschiedene Draufsichten auf die Oberfläche des Sensorgehäuses. Die Elektroden 7 und 7' sind der EKG-Einheit und der Impedanzmesseinheit der Messvorrichtung zugeordnet. Die Figur 8 zeigt die Anordnung von Gegenelektroden, die für eine Arm-zu-Arm-Messung des EKGs und der bioelektrischen Impedanz verwendet werden. Die Impedanzmesseinheit umfasst die in den Figuren 6 und 7 dargestellten Elektroden 7 zur Einspeisung von elektrischem Wechselstrom, und Messelektroden 7' zu Impedanzmessung des Körpergewebes, beispielsweise im Bereich des Fingers des untersuchten Patienten (vgl. Figur 3). Aufgrund der Vierpunktmessung verfälschen Übergangswiderstände zwischen den Elektroden 7, 7' und dem Köpergewebe die Messungen nicht. Der Abstand zwischen den Elektroden 7, 7' kann nur wenige Millimeter bis wenige Zentimeter betragen. Beim Messvorgang berühren alle vier Elektroden, die in den Figuren 6 und 7 gezeigt sind, gleichzeitig denselben Bereich der Hautoberfläche, z.B. am Finger des Patienten. Durch Einspeisung von Wechselstrom variabler Frequenz ist die Messung der komplexen Impedanz möglich. Das Messsignal wird über die Elektroden 7' mittels eines (nicht dargestellten) Spannungsmessers erfasst. Das Messsignal wird mittels eines (ebenfalls nicht dargestellten) Analog-/Digitalwandlers digitalisiert und danach einer diskreten Fouriertransformation unterzogen. Das Ergebnis liefert dann den Real- und den Imaginärteil der Impedanz, d.h. den Resistanz- und den Reaktanzwert. Bei den in den Figuren 6 und 7 dargestellten Ausführungsbeispielen sind die Elektroden 7, 7' als parallel zueinander beabstandete Streifen ausgebildet, die durch die dazwischenliegenden Zwischenräume voneinander elektrisch isoliert sind. In den Zwischenräumen sind die Lichtquellen 4, die Strahlungssensoren 5 sowie der Wärmesensor 6 angeordnet. Sämtliche Sensoren sind mit dem zu untersuchenden Körpergewebe in Kontakt.

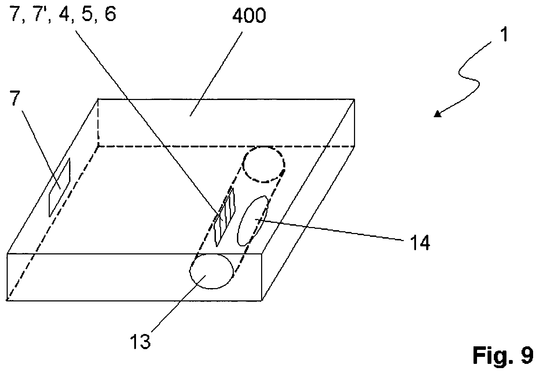

Die Figur 9 zeigt schematisch ein weiteres Ausführungsbeispiel der erfindungsgemäßen Messvorrichtung 1. An der Außenseite des Gehäuses 400 ist eine EKG-Elektrode 7 angebracht. Diese Elektrode wird mit dem Finger einer Hand berührt. Ein Finger der anderen Hand wird in eine röhrenförmige Öffnung 13 eingeführt. Im Inneren der Öffnung 13 befinden sich die Elektroden 7, 7', eine Strahlungsquelle 4, Strahlungssensoren 5 sowie Wärmesensor 6. Weiterhin ist im Inneren der Röhre 13 ein aufblasbares Luftkissen 14 angeordnet, welches den Finger fixiert und sanft und mit definiertem Druck gegen die Sensoren presst. Bedientasten der Messvorrichtung 1 sowie eine Anzeige zur Ausgabe der Messergebnisse sind aus Gründen der Übersichtlichkeit in der Figur 9 weggelassen.

Die Figur 4 zeigt einen Hohlreflektor 701, in dem als Strahlungsquelle eine (oder mehrere) LED 702 angeordnet ist. Es handelt sich um eine SMD-LED, die in alle Raumrichtungen Licht emittiert. Andere Arten von Lichtquellen sind gemäß der Erfindung ebenfalls möglich. Das Licht der LED 702 wird an den inneren Oberflächen des Hohlreflektors 701 diffus reflektiert. Der Hohlreflektor 701 ist bei dem Ausführungsbeispiel nach Art einer Ulbricht-Kugel ausgebildet. An den Hohlreflektor 701 ist eine Optik 703 in Form einer Sammellinse angebunden. Die Sammellinse bewirkt, wie in der Figur 4 angedeutet, eine gerichtete Abstrahlung des von der LED 702 abgestrahlten Lichtes. Weiterhin weist der Hohlreflektor 701 eine Austrittsöffnung 704 auf, durch welche eine diffuse Abstrahlung in das Körpergewebe erfolgt. Auch im Bereich der Austrittsöffnung 704 kann eine geeignete Optik vorgesehen sein, um den gewünschten Abstrahlwinkel zu erzielen. Unterschiedliche Abstrahlwinkel können z.B. mittels Linsen unterschiedlicher Brennweiten oder anderer optischer Elemente, wie z.B. Kollimatoren etc., erzeugt werden. Die in Figur 4 gezeigte Anordnung kann mit Vorteil als kompakte vorgefertigte Einheit zu geringen Kosten in großen Stückzahlen hergestellt werden. Die elektrischen Anschlüsse 705 der LED sind aus dem Hohlreflektor heraus geführt. Der Hohlreflektor kann z.B. als massiver Körper aus transparentem Kunststoff ausgebildet sein, in den die LED 702 eingebettet ist. Dieser kann dann an seiner Außenseite mit diffus reflektierendem Material (z.B. Aluminiumoxid oder Bariumsulfat) beschichtet sein. Entscheidend kommt es darauf an, dass durch diffuse Reflektion die Richtcharakterisitik der LED aufgehoben wird und dann, durch Auskopplung des Lichtes in geeigneter Weise aus dem Reflektor, die gewünschte Abstrahlcharakteristik nach den jeweiligen Anforderungen vorgegeben werden kann. Es ist problemlos möglich, mehrere LEDs in einem Hohlreflektor unterzubringen, um Strahlung bei verschiedenen Wellenlängen zu erzeugen und einheitlich über die an den Hohlreflektor 701 angebundene Optik 703 bzw. über die Austrittföffnung 704 in das Körpergewebe einzustrahlen.

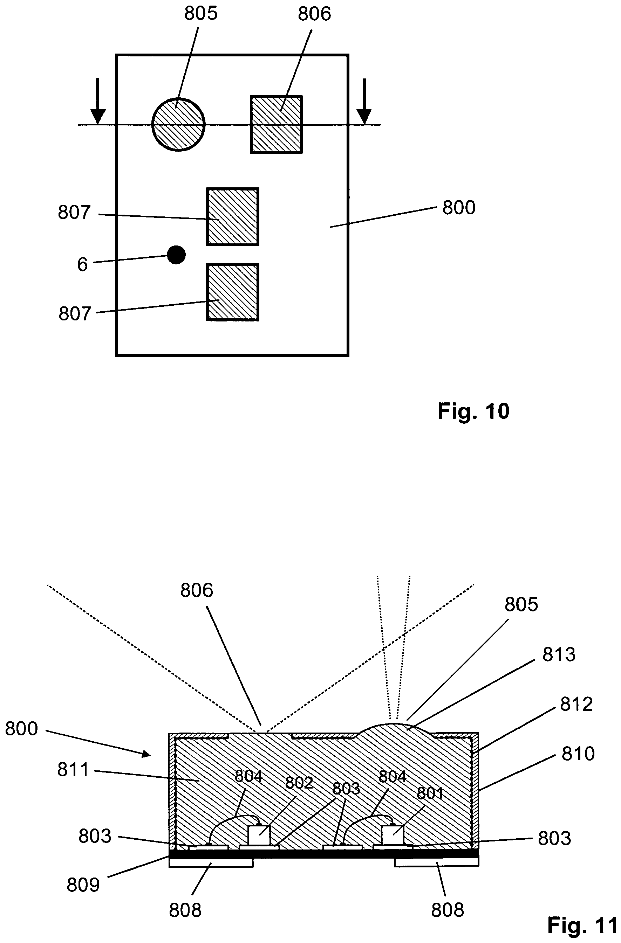

Die Figuren 10 und 11 zeigen ein Chipgehäuse 800, das einen Hohlreflektor im Sinne der Erfindung bildet. Das Chipgehäuse 800 nimmt in sich die Strahlungsquelle und den Strahlungssensor auf. Bei dem dargestellten Ausführungsbeispiel sind zwei Strahlungsquellen mit Halbleiterkörpern 801 und 802 vorgesehen, bei denen es sich z.B. um LED-Chips handelt. Im Inneren des Chipgehäuses verlaufen elektrische Leiterbahnen 803. Die elektrischen Verbindungen mit den Halbleiterkörpern 801, 802 sind über Die-Bonding bzw. über Bond-Drähte 804 hergestellt. An seiner Oberseite, die in der Figur 10 zu sehen ist, weist das Chipgehäuse 800 Durchtrittsöffnungen 805, 806 und 807 zum Austritt der von den Halbleiterkörpern 801, 802 emittierten Strahlung in das zu untersuchende Körpergewebes bzw. zum Eintritt der von dem Körpergewebe gestreuten und/oder transmittierten Strahlung in das Chipgehäuse 800 auf. Als Strahlungssensoren sind innerhalb des Chipgehäuses 800 hinter den Durchtrittsöffnungen 807 Photodioden (nicht dargestellt) angeordnet. Das Chipgehäuse 800 bildet die Ummantelung der Halbleiterkörper 801, 802 und der Photodioden. Außerdem sind an der Unterseite des Chipgehäuses 800 elektrische Anschlussstellen 808, über welche das Chipgehäuse 800 auf einer Leiterplatte (nicht dargestellt) der erfindungsgemäßen Messvorrichtung zu befestigen und mit einer Schaltung auf der Leiterplatte zu verbinden ist. Die elektrischen Leiterbahnen 803 sind auf einem Substrat 809 angeordnet, das die Unterseite des Chipgehäuses 800 bildet. Die Oberseite bildet ein Gehäusedeckel 810. Die auf dem Substrat 809 befestigten und verdrahteten Halbleiterkörper 801, 802 sind von einem transparenten Kunststoffmaterial 811 (z.B. Epoxidharz, Silikonharz, Quarzglas oder PMMA) umgeben, das das Gehäuseinnere, wie in Figur 11 zu erkennen ist, ausfüllt. Im Bereich der Durchtrittsöffnungen 805, 806, 807 weist der Gehäusedeckel 810 entsprechende Ausnehmungen auf. Auf diese Weise sind Halbleiterkörper 801, 802 nicht direkt der Umwelt ausgesetzt. Das Chipgehäuse fungiert als Hohlreflektor. Das bedeutet, dass die von den Halbleiterkörpern 801, 802 emittierte Strahlung (mehrfach) an den Innenflächen des Chipgehäuses 800 reflektiert wird. Hierzu kann das Chipgehäuse 800 an seinen inneren Wandungen mit einem diffus reflektierenden Material 812 beschichtet sein. Alternativ kann das Chipgehäuse 800 z.B. aus weißem, d.h. reflektierendem Kunststoffmaterial gefertigt sein. Die im Inneren reflektierte und gestreute Strahlung der Halbleiterkörper 801, 802 ist nahezu ideal diffus. Die Strahlung z.B. des Halbleiterkörpers 802 verlässt somit das Chipgehäuse 800 durch die Durchtrittsöffnung 806 unter einem sehr großen Abstrahlwinkel von ca. 110° in das zu untersuchende Körpergewebe. Im Bereich der Durchtrittsöffnung 805 bildet das das Chipgehäuse 800 ausfüllende transparente Kunststoffmaterial 811 eine Sammellinse 813. Die Sammellinse 813 bewirkt, wie in der Figur 11 angedeutet, eine gerichtete Abstrahlung der von dem Halbleiterkörper 801 abgestrahlten Strahlung in das Körpergewebe unter einem Winkel von ca. 10°. In das Chipgehäuse 800 kann weiterhin der Wärmesensor 6 integriert sein.

**Patentansprüche**

1.   Diagnostische Messvorrichtung zur nicht-invasiven Erfassung von wenigstens einem physiologischen Parameter von Körpergewebe, mit einer optischen Messeinheit (100), die wenigstens eine Strahlungsquelle (4, 702) zur Bestrahlung des zu untersuchenden Körpergewebes und wenigstens einen Strahlungssensor (5) zur Detektion der von dem Körpergewebe gestreuten und/oder transmittierten Strahlung umfasst, wobei die wenigstens eine Strahlungsquelle (4, 702) in einem Hohlreflektor (701) angeordnet ist,
     **dadurch gekennzeichnet,**
     **dass** die Abstrahlung des Lichts der wenigstens einen Strahlungsquelle (4, 702) in der Weise erfolgt, dass Lichtstrahlen mit unterschiedlichen räumlichen Abstrahlcharakteristiken das untersuchte Körpergewebe bestrahlen, wobei am Hohlreflektor wenigstens zwei Strahlungsaustrittsöffnungen vorgesehen sind und an wenigstens einer dieser Austrittsöffnungen eine Optik zur Veränderung der räumlichen Abstrahlcharakteristik der aus dem Hohlreflektor emittierten Strahlung angebunden ist.

2.   Diagnostische Messvorrichtung nach Anspruch 1, **dadurch gekennzeichnet, dass** verschiedene Volumenbereiche des untersuchten Körpergewebes bestrahlt werden.

3. Diagnostische Messvorrichtung nach einem der Ansprüche 1 oder 2, **dadurch gekennzeichnet, dass** eine EKG-Einheit (132) zur Erfassung eines EKG-Signals über zwei oder mehr EKG-Elektroden (7) und/oder eine bioelektrische Impedanzmesseinheit (130) mit Einspeise-und Messelektroden (7, 7') vorgesehen ist.

4. Diagnostische Messvorrichtung nach Anspruch 3, **dadurch gekennzeichnet, dass** die wenigstens eine Strahlungsquelle (4, 702) und der wenigstens eine Strahlungssensor (5) in einem gemeinsamen Sensorgehäuse (400) angeordnet sind und an der Gehäuseoberfläche des Sensorgehäuses (400) wenigstens eine Elektrode (7) angebracht ist, welche die Hautoberfläche in dem von der optischen Messeinheit (100) erfassten Bereich des Körpergewebes berührt, wobei die wenigstens eine Elektrode (7) als EKG-Elektrode und/oder Impedanzeinspeiseelektrode und/oder Impedanzmesselektrode ausgebildet ist.

5. Diagnostische Messvorrichtung nach Anspruch 3, **dadurch gekennzeichnet, dass** in oder an dem Sensorgehäuse (400) ein Temperatur- oder Wärmesensor (6) angeordnet ist.

6. Diagnostische Messvorrichtung nach Anspruch 4, **dadurch gekennzeichnet, dass** die EKG-Elektrode (7) oder die Einspeise- oder Messelektrode (7, 7') wenigstens eine Ausnehmung für den Durchtritt der von der wenigstens einen Strahlungsquelle (4) emittierten Strahlung in das zu untersuchende Körpergewebe oder für den Durchtritt der von dem Körpergewebe gestreuten und/oder transmittierten Strahlung in den Strahlungssensor (5) aufweist.

7. Diagnostische Messvorrichtung nach einem der Ansprüche 3 bis 6, **dadurch gekennzeichnet, dass** die bioelektrische Impedanzmesseinheit (130) zur Erfassung eines Impedanzmesssignals von der Hautoberfläche über wenigstens ein Messelektrodenpaar (7') eingerichtet, wobei der Elektrodenabstand des Messelektrodenpaares (7') weniger als einen Millimeter bis einige Zentimeter beträgt, in der Weise, dass beim Messvorgang beide Elektroden des Messelektrodenpaares (7') zur lokalen Erfassung des Impedanzmesssignals gleichzeitig im selben Bereich die Hautoberfläche berühren.

8. Diagnostische Messvorrichtung nach einem der Ansprüche 1 bis 7, **gekennzeichnet durch** eine Fixiervorrichtung (14) zur Fixierung eines Körperteils des zu untersuchenden Patienten an dem Sensorgehäuse (400).

9. Diagnostische Messvorrichtung nach einem der Ansprüche 1 bis 8, **gekennzeichnet durch** eine Verbindung mit einem Gerät der Unterhaltungs- oder Kommunikationstechnik oder mit einem anderweitigen tragbaren Gerät oder Accessoire.

10. Diagnostische Messvorrichtung nach einem der Ansprüche 1 bis 9, **dadurch gekennzeichnet, dass** der Hohlreflektor durch ein Chipgehäuse (800) gebildet ist, das in sich die wenigstens eine Strahlungsquelle (4) und/oder den wenigstens einen Strahlungssensor (5) aufnimmt, wobei die Strahlungsquelle (4) und der Strahlungssensor (5) jeweils wenigstens einen Halbleiterkörper (801, 802) umfassen, der über im Inneren des Chipgehäuses (800) verlaufende elektrische Leiterbahnen (803) und/oder über Bond-Drahtverbindungen (809) kontaktiert ist, wobei das Chipgehäuse (800) an seiner Oberseite Durchtrittsöffnungen (805, 806, 807) zum Durchtritt der von der Strahlungsquelle (4) emittierten Strahlung in das zu untersuchenden Körpergewebe und/oder zum Durchtritt der von dem Körpergewebe gestreuten und/oder transmittierten Strahlung umfasst.

11. Diagnostische Messvorrichtung nach einem der Ansprüche 1 bis 10, **dadurch gekennzeichnet, dass** die am Hohlreflektor (701) angebundene Optik (703, 813) eine gerichtete Abstrahlung in das Körpergewebe bewirkt.

12. Diagnostische Messvorrichtung nach den Ansprüchen 10 oder 11, **dadurch gekennzeichnet, dass** die Optik (813) durch ein das Chipgehäuse (800) ausfüllendes transparentes Kunststoffmaterial (811) gebildet ist.

13. Diagnostische Messvorrichtung nach einem der Ansprüche 1 bis 12, **dadurch gekennzeichnet, dass** durch eine der Austrittsöffnungen (704, 806) des Hohlreflektors (701) eine diffuse Abstrahlung in das Körpergewebe erfolgt.

**Claims**

1. Diagnostic measurement apparatus for the non-invasive capture of at least one physiological parameter of body tissue, having an optical measurement unit (100) comprising at least one radiation source (4, 702) for irradiating the body tissue to be examined and at least one radiation sensor (5) for detecting the radiation that was scattered and/or transmitted by the body tissue, wherein the at least one radiation source (4, 702) is arranged in a concave reflector (701),

**characterized**

**in that** the light of the at least one radiation source (4, 702) is emitted in such a way that light rays with different spatial emission characteristics irradiate the examined body tissue, wherein at least two radiation exit openings are provided at the concave reflector and an optical unit for modifying the spatial emission characteristic of the radiation emitted from the concave reflector is attached to at least one of these exit openings.

2. Diagnostic measurement apparatus according to Claim 1, **characterized in that** different volume regions of the examined body tissue are irradiated.

3. Diagnostic measurement apparatus according to either of Claims 1 and 2, **characterized in that** provision is made of an ECG unit (132) for capturing an ECG signal by way of two or more ECG electrodes (7) and/or of a bioelectric impedance measurement unit (130) with supply and measurement electrodes (7, 7').

4. Diagnostic measurement apparatus according to Claim 3, **characterized in that** the at least one radiation source (4, 702) and the at least one radiation sensor (5) are arranged in a common sensor housing (400) and at least one electrode (7), which touches the skin surface in the region of the body tissue captured by the optical measurement unit (100), is attached to the housing surface of the sensor housing (400), wherein the at least one electrode (7) is embodied as an ECG electrode and/or impedance supply electrode and/or impedance measurement electrode.

5. Diagnostic measurement apparatus according to Claim 3, **characterized in that** a temperature or heat sensor (6) is arranged in or on the sensor housing (400) .

6. Diagnostic measurement apparatus according to Claim 4, **characterized in that** the ECG electrode (7) or the supply or measurement electrode (7, 7') has at least one cut-out for the passage of the radiation emitted by the at least one radiation source (4) into the body tissue to be examined or for the passage of the radiation that was scattered and/or transmitted by the body tissue into the radiation sensor (5).

7. Diagnostic measurement apparatus according to any one of Claims 3 to 6, **characterized in that** the bioelectric impedance measurement unit (130) is configured to capture an impedance measurement signal from the skin surface by way of at least one measurement electrode pair (7'), wherein the electrode spacing of the measurement electrode pair (7') is from less than one millimetre to a few centimetres in such a way that, during the measurement process, both electrodes of the measurement electrode pair (7') touch the skin surface simultaneously in the same region for the local capture of the impedance measurement signal.

8. Diagnostic measurement apparatus according to any one of Claims 1 to 7, **characterized by** a fixation apparatus (14) for fixing a body part of the patient to be examined on the sensor housing (400).

9. Diagnostic measurement apparatus according to any one of Claims 1 to 8, **characterized by** a link to a device from the entertainment or communications technology sector or to another portable device or accessory.

10. Diagnostic measurement apparatus according to any one of Claims 1 to 9, **characterized in that** the concave reflector is formed by a chip housing (800) that receives the at least one radiation source (4) and/or the at least one radiation sensor (5) therein, wherein the radiation source (4) and the radiation sensor (5) each comprise at least one semiconductor body (801, 802) that is contacted via electrical conductor tracks (803) running in the interior of the chip housing (800) and/or by way of bonding wire connections (809), wherein the chip housing (800) comprises, on its upper side, passage openings (805, 806, 807) for the passage of the radiation emitted by the radiation source (4) into the body tissue to be examined and/or for the passage of the radiation that was scattered and/or transmitted by the body tissue.

11. Diagnostic measurement apparatus according to any one of Claims 1 to 10, **characterized in that** the optical unit (703, 813) attached to the concave reflector (701) brings about a targeted emission into the body tissue.

12. Diagnostic measurement apparatus according to Claim 10 or 11, **characterized in that** the optical unit (813) is formed by a transparent plastics material (811) that fills the chip housing (800).

13. Diagnostic measurement apparatus according to any one of Claims 1 to 12, **characterized in that** there is diffuse emission into the body tissue through one of the exit openings (704, 806) of the concave reflector (701) .

**Revendications**

1. Dispositif de mesure de diagnostic pour l'acquisition non invasive d'au moins un paramètre physiologique de tissus organiques, comprenant une unité de mesure optique (100) qui comporte au moins une source de rayonnement (4, 702) destinée à irradier le tissu organique à examiner et au moins un détecteur de rayonnement (5) destiné à détecter le rayonnement diffusé et/ou transmis par le tissu organique, l'au moins une source de rayonnement (4, 702) étant disposée dans un réflecteur creux (701), **caractérisé en ce que** l'émission de la lumière de l'au moins une source de rayonnement (4, 702) s'effectue de telle sorte que des rayons lumineux ayant des caractéristiques de rayonnement dans l'espace différentes irradient le tissu organique examiné, au moins deux ouvertures de rayonnement étant présentes sur le réflecteur creux et une optique destinée à modifier la caractéristique de rayonnement dans l'espace du rayonnement émis depuis le réflecteur creux étant reliée à au moins l'une de ces ouvertures de sortie.

2. Dispositif de mesure de diagnostic selon la revendication 1, **caractérisé en ce que** différentes zones du volume du tissu organique examiné sont irradiées.

3. Dispositif de mesure de diagnostic selon l'une des revendications 1 ou 2, **caractérisé par** la présence d'une unité d'ECG (132) destinée à acquérir un signal d'ECG par le biais d'au moins deux électrodes d'ECG (7) et/ou d'une unité de mesure d'impédance bioélectrique (130) comprenant des électrodes d'injection et de mesure (7, 7').

4. Dispositif de mesure de diagnostic selon la revendication 3, **caractérisé en ce que** l'au moins une source de rayonnement (4, 702) et l'au moins un détecteur de rayonnement (5) sont disposés dans un boîtier de détecteur (400) commun et au moins une électrode (7) est montée sur la surface du boîtier de détecteur (400), laquelle entre en contact avec la surface de la peau dans la zone du tissu organique captée par l'unité de mesure optique (100), l'au moins une électrode (7) étant réalisée sous la forme d'une électrode d'ECG et/ou d'une électrode d'injection à impédance et/ou d'une électrode de mesure d'impédance.

5. Dispositif de mesure de diagnostic selon la revendication 3, **caractérisé en ce qu'**un détecteur de température ou de chaleur (6) est disposé dans ou sur le boîtier de détecteur (400).

6. Dispositif de mesure de diagnostic selon la revendication 4, **caractérisé en ce que** l'électrode d'ECG (7) ou l'électrode d'injection et de mesure (7, 7') possède au moins une cavité pour le passage du rayonnement émis par l'au moins une source de rayonnement (4) dans le tissu organique à examiner ou pour le passage du rayonnement diffusé et/ou transmis par le tissu organique dans le détecteur de rayonnement (5).

7. Dispositif de mesure de diagnostic selon l'une des revendications 3 à 6, **caractérisé en ce que** l'unité de mesure d'impédance bioélectrique (130) est conçue pour acquérir un signal de mesure d'impédance de la surface de la peau par le biais d'au moins une paire d'électrodes de mesure (7'), l'espacement des électrodes de la paire d'électrodes de mesure (7') étant de un millimètre à quelques centimètres, de sorte que lors de l'opération de mesure, les deux électrodes de la paire d'électrodes de mesure (7') entrent en contact simultanément dans la même zone de la surface de la peau en vue d'acquérir le signal de mesure d'impédance.

8. Dispositif de mesure de diagnostic selon l'une des revendications 1 à 7, **caractérisé par** un dispositif de fixation (14) destiné à fixer une partie du corps du patient à examiner au boîtier de détecteur (400).

9. Dispositif de mesure de diagnostic selon l'une des revendications 1 à 8, **caractérisé par** une liaison avec un appareil de la technologie de divertissement ou de communication ou avec un autre appareil ou accessoire portable.

10. Dispositif de mesure de diagnostic selon l'une des revendications 1 à 9, **caractérisé en ce que** le réflecteur creux est formé par un boîtier de puce (800) qui accueille à l'intérieur de celui-ci l'au moins une source de rayonnement (4) et/ou l'au moins un détecteur de rayonnement (5), la source de rayonnement (4) et le détecteur de rayonnement (5) comportant respectivement un corps en semiconducteur (801, 802) avec lequel le contact est établi par le biais de pistes conductrices électriques (803) et/ou de liaisons par fil de connexion (809) qui s'étendent à l'intérieur du boîtier de puce (800), le boîtier de puce (800) comportant sur son côté supérieur des ouvertures de passage (805, 806, 807) destinées au passage du rayonnement émis par la source de rayonnement (4) dans le tissu organique à examiner et/ou au passage du rayonnement diffusé et/ou transmis par le tissu organique.

11. Dispositif de mesure de diagnostic selon l'une des revendications 1 à 10, **caractérisé en ce que** l'optique (703, 813) reliée au réflecteur creux (701) produit un rayonnement dirigé dans le tissu organique.

12. Dispositif de mesure de diagnostic selon l'une des revendications 10 ou 11, **caractérisé en ce que** l'optique (813) est formée par une matière plastique transparente (811) qui remplit le boîtier de puce (800).

13. Dispositif de mesure de diagnostic selon l'une des revendications 1 à 12, **caractérisé en ce qu'**un rayonnement diffus dans le tissu organique a lieu à travers l'une des ouvertures de sortie (704, 806) du réflecteur creux (701).

SaO2: 97
StO2: 70
SvO2: 65
HR: 68
BF: 1.2

BG: 90

**Fig. 1**

| 100 | | | | |
| 120 | 110 | 140 | 150 | 160 |
| 130 | | | | 170 |
| 132 | | | | 180 |

**Fig. 2**

**Fig. 3**

**Fig. 4**

**Fig. 5**

**Fig. 6**

**Fig. 7**

**Fig. 8**

Fig. 9

805    806

807

6

807                                                    800

**Fig. 10**

806                                    805

                                              813

                                              812

800
                                              810
811

804  802  803  804  801

803                                            803

809

808                                808

**Fig. 11**

## IN DER BESCHREIBUNG AUFGEFÜHRTE DOKUMENTE

### In der Beschreibung aufgeführte Patentdokumente

- US 6041247 A

### In der Beschreibung aufgeführte Nicht-Patentliteratur

- **MEIR NITZAN ; BORIS KHANOKH.** Infrared Radiometry of Thermally Insulated Skin for the Assessment of Skin Blood Flow. *Optical Engineering,* 1994, vol. 33 (9), 2953-2956